# EUROPEAN PATENT SPECIFICATION

(11) **EP 2 349 366 B1**
(45) Date of publication and mention of the grant of the patent: **28.08.2013**
(21) Application number: 09816970.9
(22) Date of filing: 28.09.2009
(51) Int. Cl.: A61L 27/36, A61F 2/02

(54) **ACTIVE SILK MUCO-ADHESIVES, SILK ELECTROGELATION PROCESS, AND DEVICES**
AKTIVE SEIDEN-MUKOADHÄSIVA, SEIDEN-ELEKTROGELATIONSVERFAHREN UND VORRICHTUNGEN
MUCO-ADHÉSIFS ACTIFS EN SOIE, PROCÉDÉ ET DISPOSITIFS D'ÉLECTROGÉLIFICATION DE LA SOIE

(30) Priority: 26.09.2008 US 100352 P; 03.03.2009 US 156976 P
(43) Date of publication of application: 03.08.2011
(60) Divisional application: 13175299.0
(73) Proprietor: Trustees of Tufts College, Medford, MA 02155 (US)
(72) Inventor: BERNHARD, Nicolai N., CH-8400 Winterthur (CH); LO, Tim Jia-Ching, Somerville Massachusetts 02144 (US); LEISK, Gary G., Wilmington Massachusetts 01887 (US); KAPLAN, David L., Concord Massachusetts 01742 (US)
(74) Representative: Purdylucey Intellectual Property
(86) International application number: PCT/US2009/058534
(87) International publication number: WO 2010/036992

(56) References cited:
- WO-A2-2005/012606
- JP-A- 4 263 611
- US-A1- 2004 266 992
- US-A1- 2005 260 706
- US-A1- 2007 187 862
- US-A1- 2008 085 272
- ANDO Y: "Piezoelectric and Related properties of Hydrated Silk Fibroin", REPORTS ON PROGRESS IN POLYMER PHYSICS IN JAPAN, GAKUJUTSU BUNKEN FUKYUKAI, TOKYO, JP, vol. XXIII, 9 October 1980 (1980-10-09), pages 775-778, XP003001687, ISSN: 0486-4476

## Description

### FIELD OF THE INVENTION

The present invention provides for methods and devices for rapidly and reversibly converting silk fibroin solution into a gel by applying an electric field.

### BACKGROUND

Silk is generally defined as protein polymers spun into fibers by some *Lepidoptera* larvae such as silk worms and spiders. Silk is an intriguing biomaterial because of its light weight, incredible strength, and elasticity. The major components of silkworm *Bombyx mori* silk are fibroin protein and glue-like sericin protein. Silk fibroin protein compositions are attractive in biomedical applications, such as tissue engineering, because of their biocompatibility, slow degradability, and excellent mechanical properties. Degradable silk fibroin formulations offer mechanically robust materials for a wide range of mechanical and functional properties for biomedical applications including drug delivery. Among the useful silk fibroin preparations are silk gels. There remains a need for additional approaches to forming silk gels for a variety of applications.

### SUMMARY OF THE INVENTION

The embodiments of the present invention provide for methods of reversibly converting solubilized silk fibroin solution into a silk fibroin gel by applying an electric field to the silk fibroin solution in a process called electrogelation. Applying an electric field to the solubilized silk fibroin solution may be performed, for example, by submerging electrodes in a solution of solubilized silk fibroin and applying a constant voltage across the electrodes. The electric field created by the voltage causes a conformational change of the silk fibroin from random coil to the silk I formation. Although voltages of up to about 50 VDC are effective in inducing gelation, higher voltages may be useful. In general, unless the silk fibroin solution has additional additives such as salt that reduce its electrical resistance, the voltage application may be performed at low current. In testing below about 50 VDC, the typical current is below about 10 milliamps. With the addition of salt to the solubilized silk fibroin solution, however, the current is increased dramatically, above about 80 milliamps, accompanied by increased gelation rates. When the DC voltage is reversed (reversal of the electrode polarity), the silk gel tends to convert back to a liquid form (random coil conformation).

Electrogelation has advantages over other mechanisms of inducing silk gelation, such as pH manipulation, heating, application of various ions and mechanical shearing, for several reasons. For example, an increase in the meta-stable silk I conformation of the silk fibroin achieved with electrogelation provides a more versatile material phase compared with β-sheet conformation generated by other gelation techniques. Additionally, the silk I structures exhibited in gel form can be transformed back to liquid form (random coil form); or through minimal additional molecular alignment, can be converted to β-sheet structures. This ability is an important characteristic of "smart structures."

Electrogelation can also be applied using a wide range of electrode materials and geometries, from platinum wire electrodes to mating metal plates to tissues (*in vitro* or *in vivo*) that can be coated with silk gel. Because a simple voltage need be applied, for example DC voltage from a battery-operated device, as opposed to other chemical manipulations, the process may be more benign in biomedical applications where living cells might be impacted.

An aspect of the present invention also provides for a piezoelectric silk material comprising a mixture of silk fibroin proteins having silk I conformation and silk β-sheet structure. The piezoelectric property of this silk material is characterized by the ratio of silk I conformation and silk β-sheet structure, which can be determined by the applying the methods presented herein. For example, the content of silk I conformation is adjusted by DC voltage and the content of silk β-sheet structure is effected by treating the electrogelated silk with dehydration, mechanical force, or thermodynamic treatment.

Additionally, various processing and delivery devices can be envisioned easily with electrogelation. A device has been created, as described herein, that processes and delivers electrogelated silk for biomedical and non-biomedical applications where either slow large-volume delivery, or fast spray and coating delivery is desired.

The electrogelation approach described herein has tremendous potential in various application areas, from toys to firefighting to medical procedures. The process itself is easy to implement, with various manifestations envisioned.

For example, one embodiment provides for a hand-held dispenser that allows a child to safely eject a rapid stream of electrogelated silk gel. This silk gel is edible, and can be flavored or colored, and also has the ability to stick to walls but remains biodegradable; features that make it an attractive alternative to the SILLY STRING^{®} party novelty.

Silk gel generated by an electrogelation process of the present invention may also be used as biodegradable and biocompatible coatings on tissues or regenerated tissues (*in vivo* or *in vitro*) or medical devices such as medical implants. Such biodegradable and biocompatible implant coatings are of clinical interest due to their ability of continuously eluting desired drugs (e.g. anti inflammatory medication or growth factors) to the surrounding tissue of the implant.

Another embodiment provides for a hand-held device having a replaceable cartridge of silk solution. The cartridge may also contain specific cells or bioactive molecules that may be entrained in the silk solution. In the hands of a surgeon, silk gel can be ejected from the device in a coherent, slowly extruded stream to fill void space. In tissue reconstruction, for example, the gelated silk could be used to fill voids and encourage tissue growth.

Another embodiment provides for "soft robotics" in which the reversible electrogelation of silk solution provides a means of motion associated technology (e.g., camera). In particular, silk fibroin solution is not sticky, while silk gel is. This mucoadhesion characteristic mimics other natural mucoadhesives such as snail slime.

### BRIEF DESCRIPTION OF THE DRAWINGS

Figure 1 presents a schematic of electrogelation processes.

Figure 2 shows several features of silk electrogelation.

Figure 3 presents various delivery styles, properties, and additional features of silk electrogelation.

Figure 4 presents a schematic of a parallel electrode electrogelation setup.

Figure 5 shows images of parallel electrode electrogelation experiment after 0 min, 20 min and 275 min of 22.2 VDC application.

Figure 6 illustrates the silk fibroin solution electrogelation process.

Figure 7 shows images of parallel electrode electrogelation experiment after 0 min, 15 min, and 30 min of 74.9 VDC application.

Figure 8 depicts a comparison of parallel and ring electrode setups after 30 min exposure at 24.8 VDC.

Figure 9 shows a schematic of individual and dual aluminum tube electrode setups.

Figure 10 shows the results of syringe-based (3 ml) electrogelation utilizing spring-shaped electrode after 0 min, 5 min, and 15 min.

Figure 11 shows a comparison of electrogelation outcomes between 13-day-old and 65-day-old solubilized silk after 0 min and 10 min of 24.8 VDC voltage application.

Figure 12 shows the results of syringe-based (3 ml) electrogelation utilizing spring-shaped electrode after 0 min, 5 min, and 10 min of 24.8 VDC voltage application.

Figure 13 depicts electrogelation using parallel copper coil electrodes after 0 min, 5 min, and 10 min of 24.8 VDC voltage application.

Figure 14 shows the results of electrogelation of 4% and 8% solubilized silk with salt and DMEM using parallel electrode geometry with gelation times of 0 min, 5 min, and 10 min at 24.8 VDC voltage.

Figure 15 is the electrogelation of solubilized silk at 24.8 VDC voltage using graphite electrodes.

Figure 16 is a drawing of a high voltage electrogelation setup.

Figure 17 is a schematic of high voltage electrogelation results.

Figure 18 shows a mixing-electrogelation device having two electrodes with auger between them, and gelation outcome.

Figure 19 shows the Instron® materials testing device (Grove City, PA) setup for extrusion, and a representative outcome.

Figure 20A and 20B are before and after photographs of platinum electrode gelations with silk solution and DMEM.

Figures 21A-21C show the results after reversing the electrode polarity such that the silk gel returns to liquid form.

Figure 22 is a photograph of an embodiment of a hand-held silk gel extruder with the following components labeled as follows: housing 99, hydraulic pump 10, process button 11, operation valve 12, silk cartridge 13, timing and logic board 14, rechargeable batteries 15, extrusion button 16, hydraulic drive 17.

Figure 23 shows the left and right perspectives of the delivery end of an embodiment for the delivery of silk gel to a particular site. The photographs are labeled as follows: nozzle attachment 20, valve and (-) platinum electrode 21, silk solution 22, (+) platinum electrode 23.

Figure 24 is a schematic showing structural examples of piezoelectric silk-based proteins that may be created and manipulated using electrogelation.

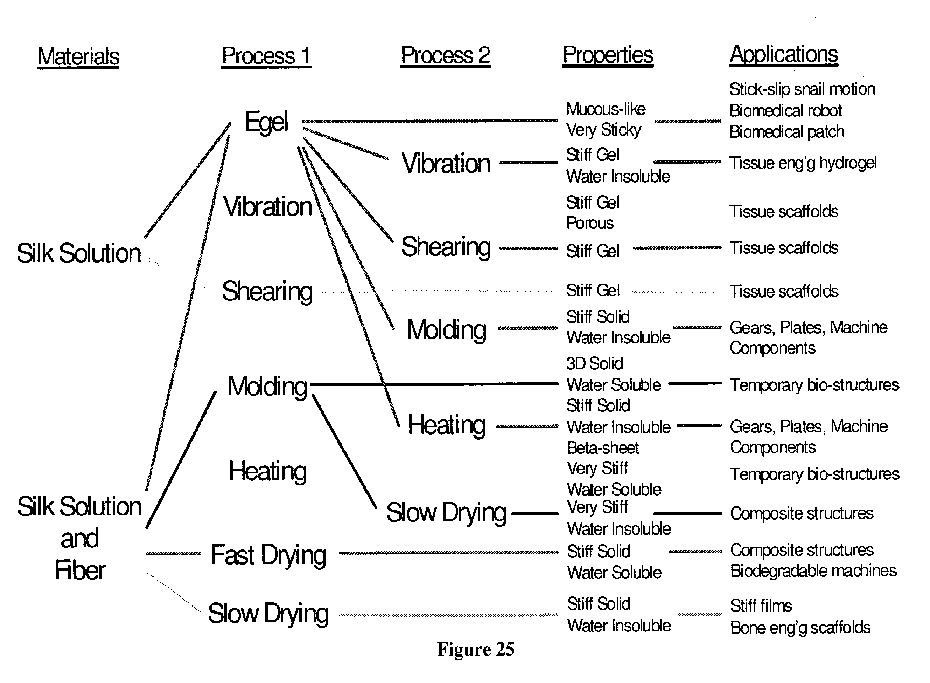

Figure 25 is a depiction of silk processing techniques, predicted properties, and potential applications.

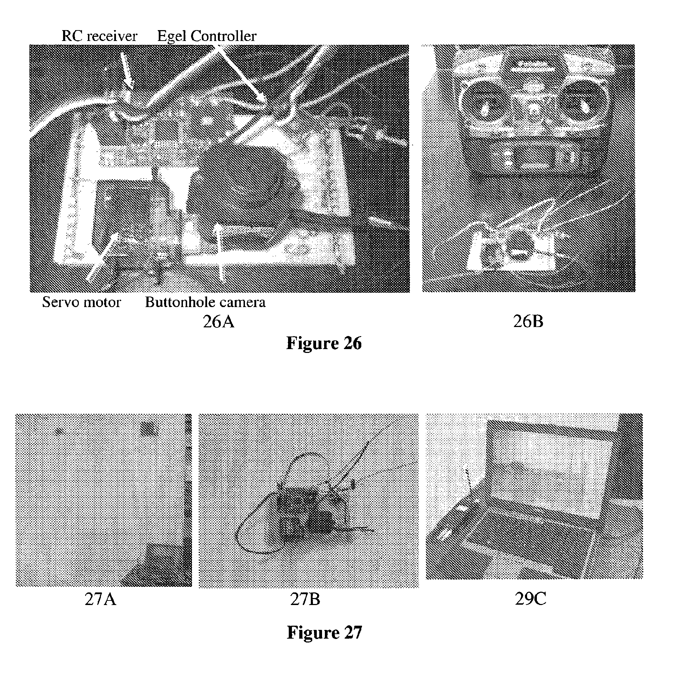

Figures 26A and 26B show components of a surveillance platform that is mobilized by silk electrogelation.

Figures 27A-27C show images from the testing of the surveillance platform.

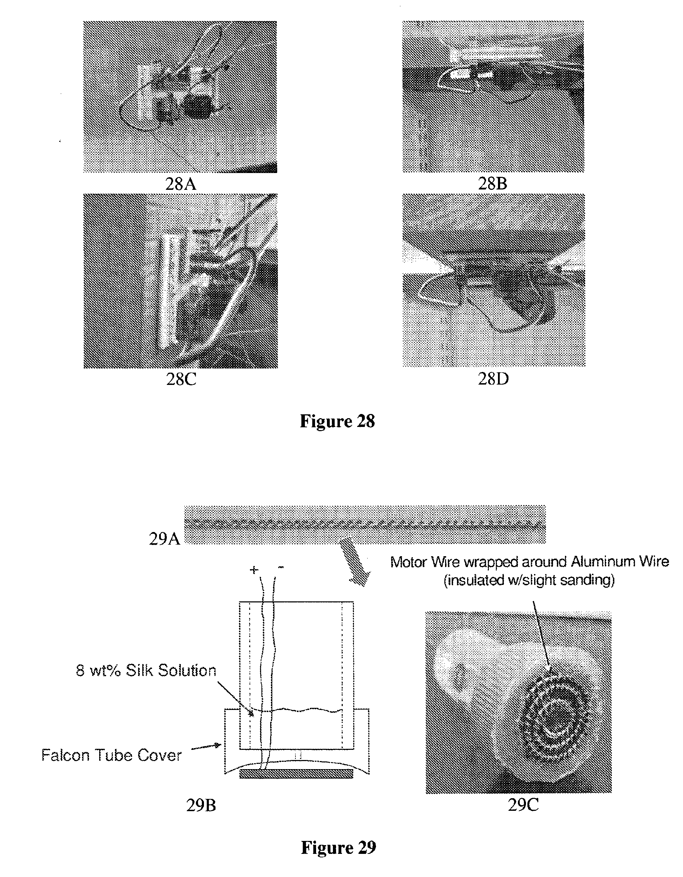

Figures 28A-28C show a surveillance platform mounted to acrylic and wood surfaces in both vertical and horizontal orientations through action of active silk muco-adhesive.

Figures 29A-29C show a helical coil electrogelation pad design.

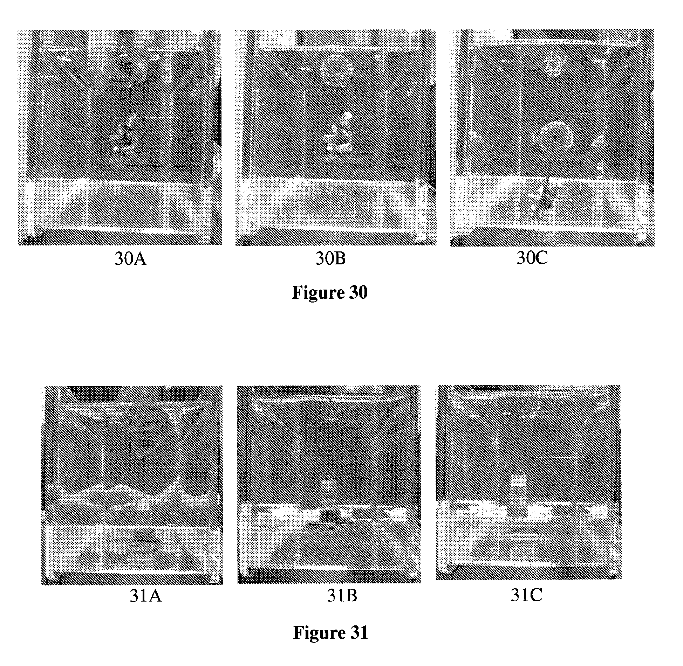

Figures 30A-30C show the electrogelation, controlled release from an underwater, vertical, Plexiglass surface.

Figures 31A- 31C show the electrogelation and controlled release as applied to the lifting of an underwater weight.

Figures 32A and 32B are pictures of a dental implant coated with the electrogelated gel before and after being dried for about 2 hours at room temperature.

### DETAILED DESCRIPTION

The terminology used herein is for the purpose of describing particular embodiments, which is defined solely by the claims.

As used herein and in the claims, the singular forms include the plural reference and vice versa unless the context clearly indicates otherwise. Other than in the operating examples, or where otherwise indicated, all numbers expressing quantities of ingredients or reaction conditions used herein should be understood as modified in all instances by the term "about."

Unless defined otherwise, all technical and scientific terms used herein have the same meaning as those commonly understood to one of ordinary skill in the art to which this invention pertains. Although any known methods, devices, and materials may be used in the practice or testing of the invention, the methods, devices, and materials in this regard are described herein.

Silk fibroin is a unique biopolymer that can be reconfigured from its native or synthesized states in various shapes and conformations. Silk fibroin protein has recently found uses well beyond textile and medical suture applications that have been the main modes of utilization in the past. For example, the generation of hydrogels (WO2005/012606; PCT/US08/65076), ultrathin films (WO2007/016524), thick films, conformal coatings (WO2005/000483; W02005/123114), microspheres (PCT/US2007/020789), 3D porous matrices (WO2004/062697), solid blocks (WO2003/056297), microfluidic devices (PCT/US07/83646; PCT/US07/83634), electro-optical devices (PCT/US07/83639), and fibers with diameters ranging from the nanoscale (WO2004/0000915) to several centimeters (U.S. Patent No. 6,902,932,) have been explored with implications in biomaterials and regenerative medicine (WO2006/042287; U.S. Patent Application Pub. No. 20070212730; PCT/US08/55072). The toughness of this natural fiber, unmatched in nature, confers impressive mechanical properties (both tensile and compressive) to silk-based materials which rival, if not exceed, most organic counterparts such as Kevlar^{®} or other polymeric materials.

Silk has been used in biomedical applications for many years. Applications range from suture materials to tissue scaffolds used in the development of engineered tissues in the body, such as tendons, cartilage and ligaments. The forms of the silk required for particular applications vary. Much research, therefore, has gone into the development of silk films (Jin et al., 15 Adv. Funct. Matter 1241-47 (2005)), non-woven mats (Jin et al., 25 Biomats. 1039-47 (2004)), sponges (porous scaffolds) (Karageorgiou et al., Part A J. Biomed. Mats. Res. 324-34 (2006)), gels (Wang et al, 29 Biomats. 1054-64 (2008)), and other forms (Sofia et al., 54 J. Biomed. Materials Res. 139-48 (2000)).

For each of these forms, insect-derived silk is usually processed into solution using a two-stage procedure. In the case of silkworm silk, cocoons from *Bombyx mori* silkworms are boiled in an aqueous solution and subsequently rinsed to remove the glue-like sericin protein that covers the natural silk. The extracted silk fibroin is then solubilized (i.e., dissolved) in LiBr before being dialyzed in water. The solubilized silk fibroin concentration can then be adjusted according to the intended use. *See* U.S. Patent Application Pub. No. 20070187862. Alternatively, recombinant silk proteins may be used. These have proved advantageous when using spider silk because arachnid-derived silk proteins are often more difficult to collect in quantity. Kluge et al., 26(5) Trends Biotechnol. 244-51 (2008). Moreover, recombinant silk fibroin may be engineered to express heterologous proteins or peptides, such as dentin matrix protein 1 and RGD, providing additional biofunctionality to the silk fibroin proteins. Huang et al., 28(14) Biomats. 2358-67 (2007); Bini et al., 7(11) Biomacromolecules 3139-45 (2006).

The processing of silk solution into a target material form requires target-specific approaches that depend on the desired geometry and morphology. For example, electrospinning is a process used to generate nano-sized fibers that can be laid up into various mat and tubular structures. In this process a high voltage potential, typically between 10 kV - 20 kV, is used to drive rapid ejection of a jet of silk solution from a needle, causing accelerated solidification of the jet into a sub-micron diameter solid fiber. The process can build up a layer with textile-like morphology and a smooth deposition underlayer. Li et al., 27 Biomats. 3115-24 (2006).

Another material form of special interest is silk gel. In biomedical applications, silk hydrogels are used for the encapsulation and delivery of cells and bioactive polymers. The high water content and mechanical response of hydrogels makes them suitable for some tissue restoration applications. In various polymer systems, a hydrogel is formed when the polymer chains cross-link into networks through chemical or physical means. Chemical triggers, such as cross-linking reagents, or physical stimulants, such as pH or temperature, have shown to be successful. Wang et al., 2008.

The silk electrogelation process as provided herein is very simple, involving the conversion of random-coil silk solution into a gel with an increase in the metastable silk I conformation by the application of an electric field using a voltage source, such as a DC or AC voltage source. Other methods of applying an electric field to the silk solution may also be used, such as current sources, antennas, lasers, and other generators. Interestingly, electrogelation using the present methods is reversible. When the DC voltage is reversed (reversal of the electrode polarity), the silk gel tends to convert back to a random coil conformation. The reversing process appears to be nearly complete, reversing can be repeated many times, and, perhaps most importantly, the nature of the material changes dramatically when alternating from gel to liquid silk solution. The gel has a very sticky, thick, mucus-like consistency. The liquid silk solution is not sticky at all and has a very low viscosity compared to the gel. The electrogelated silk, therefore, can be seen as an active silk muco-adhesive, analogous to the slime secretion that snails utilize for motion and for securing themselves to various surfaces.

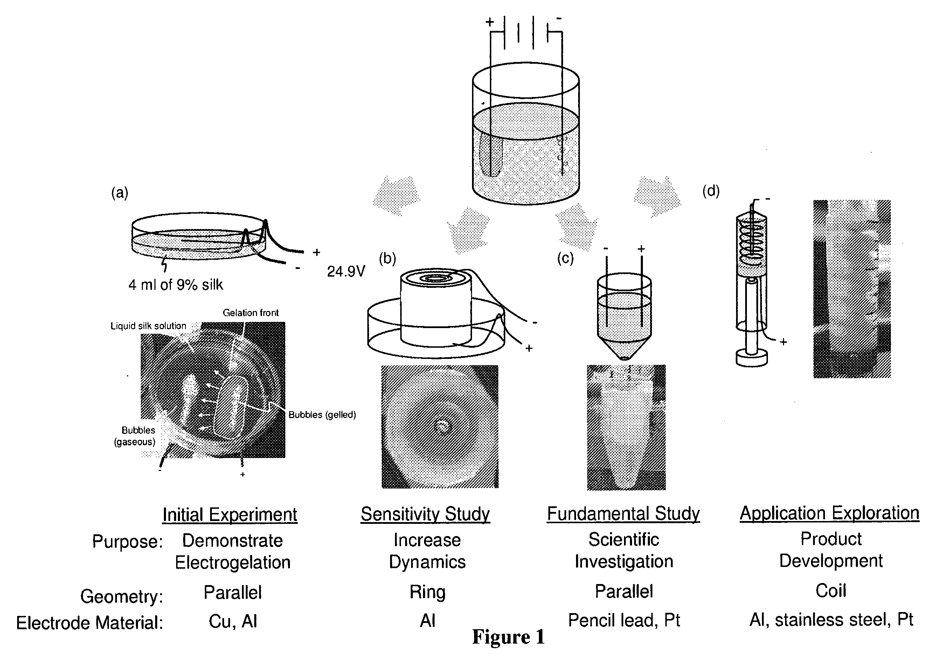

In a simple silk electrogelation example, two parallel wires (electrodes) are placed along the bottom of a 70 mm diameter plastic culture dish that contains a few ml (4 ml) of ∼9% solubilized silk fibroin solution, as shown in Figure 1A. The liquid level may slightly cover the top of the electrodes and the electrodes, which in this instance were about 40 mm apart. A voltage potential of 22.2 VDC was applied using a high-current lithium polymer (Li-Pol) battery. The electrode connected to the positive terminal of the battery is denoted "+" (the anode) and the other electrode, connected to the negative terminal of the battery is denoted "-" (the cathode). As illustrated in the photograph of Figure 1A, bubbles formed on both the positive and negative electrodes almost immediately after voltage application. The bubbles on the positive electrode, likely oxygen gas, soon became encapsulated in a growing "gel front" that emanated from the electrode outward, with a general directionality toward the negative electrode. Over the span of 275 minutes, a significant volume of the silk solution was gelated. This demonstrates the ability of the applied DC voltage to cause silk fibroin gelation. The effects of electrode surface area (Figure 1B) and material (Figure 1C) were also investigated. Other approaches, e.g., using coiled electrode geometries and/or using platinum as the electrode material (Figure 1D) also resulted in silk fibroin gelation.

The power source of the electrogelation process can be any power source that provides a direct current voltage. Direct current is produced by sources such as batteries, thermocouples, solar cells, etc. Alternatively, alternating current (AC), the general powder source for business and residence, may also be used to induce the electrogelation process, although the gel formation may not be as fast as the gelation process induced by direct current voltage.

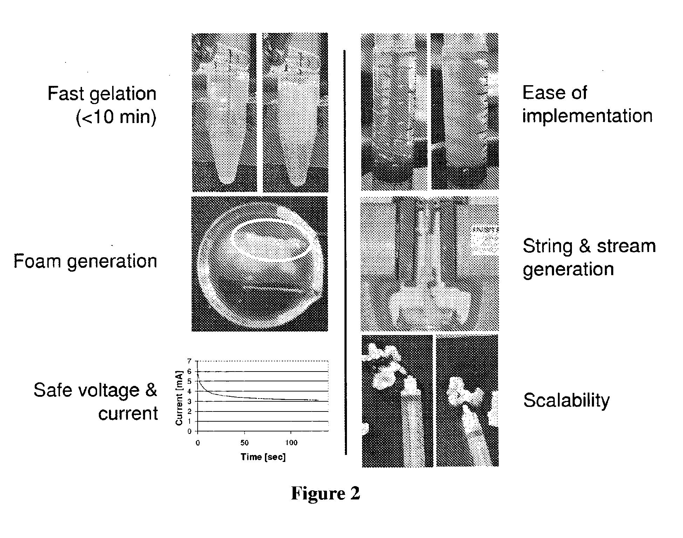

In general, after removal of the gelated silk from an electrogelation process, the resulting material is highly viscous, soft, and very tacky. Over time, the gelated silk may become very stiff. Figure 2 shows several features of silk electrogelation. Electrogelation is a low voltage and current process and can cause gelation in less than 10 minutes. The process can be incorporated in hand-held devices, providing a low cost yet scalable approach to varying target gelation volumes. Modifications of the silk can impart flavor for some applications and allow the encapsulation of cells or bioactive molecules for others. The demonstrated ability to provide biocompatibility and controlled degradation of silk are also elements that can be incorporated in application-specific manifestations.

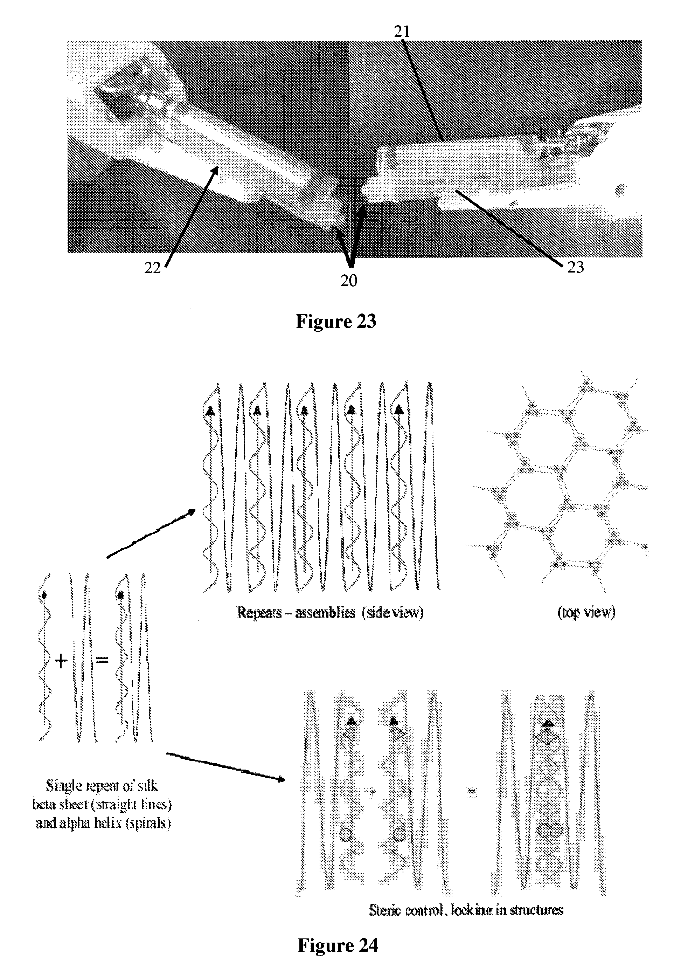

As noted, electrogelation appears to trigger a conformational shift of at least some of the silk fibroin from random coil to the silk I conformation. This phenomenon is unique from that achieved by some other mechanisms of inducing silk gelation, such as pH manipulation, heating, application of various ions and mechanical shearing, which tend to result primarily in β-sheet conformation. The increase in meta-stable silk I conformation achieved with electrogelation is a more versatile material phase to achieve than is the beta-sheet conformation. For example, the silk I gel form can be transformed back to liquid form (random coil); or through minimal additional molecular alignment, can be converted to β-sheet. This allows for the creation and manipulation of complexes comprising both structures, as shown in Figure 24.

Interestingly, silk polypeptide chains in an α-helical arrangement have also been shown to exhibit piezoelectric properties. Piezoelectric materials are known to be as high as 75% efficient in converting an electrical signal into a physical dimension change, or vice versa. Bone exhibits piezoelectric properties due to a calcified (helical) collagen matrix, which comprises much of its structure. The minute electrical signals caused by the mechanical loading and unloading of bones plays an important role in both bone formation and repair. Matching these native properties in an implanted device has the potential to speed patient recovery and aid in the restoration of pre-injury motility. A hybrid material containing the strong and slow-degrading silk motifs along oriented silk I domains provides a strong "smart" materials platform that can be used in a number of biomedical applications including improved bone repair. By tailoring the relative ratios of α-helical content to β-sheet content, the strength, degradability, and piezoelectric properties of a silk macrostructures can be varied over a range of material properties.

Without being bound by theory, electrogelation might be explained as a conformation change due to an applied electric field. Although the typical introduction of electrodes and voltage directly in the water-based silk solution instigates electrolysis, it is not thought to play a major role in the electrogelation. Electrolysis quickly affects the pH level of the silk solution at the electrodes: the pH near the positive electrodes decreases (below pH 4), while it increases near the negative electrode (above pH 13). It is likely that oxygen gas is generated at the positive electrode and hydrogen gas at the negative, influencing the pH levels. Importantly, these pH changes do ***not*** appear to be a requirement for electrogelation, which occurs independent of pH in the silk fibroin. Electrogelation may be achieved at pH ranges of about pH 6 to about pH 7.5. In terms of an electrical explanation of the process, it is important to recognize that the silk protein consists of highly repetitive amino acid sequences with small side chains (glycine, alanine, serine). In the presence of an electric field, polar side chains, which act as magnetic dipoles, may orient in the direction of the field and molecular conformations that have large electric moments perhaps increase in concentration. In the case of electrogelation, it is likely that the conformation change represented by the gel formation is due to such a mechanism. Neumann, 47 Prog. Biophys. Molec. Bio. 197-231 (1986).

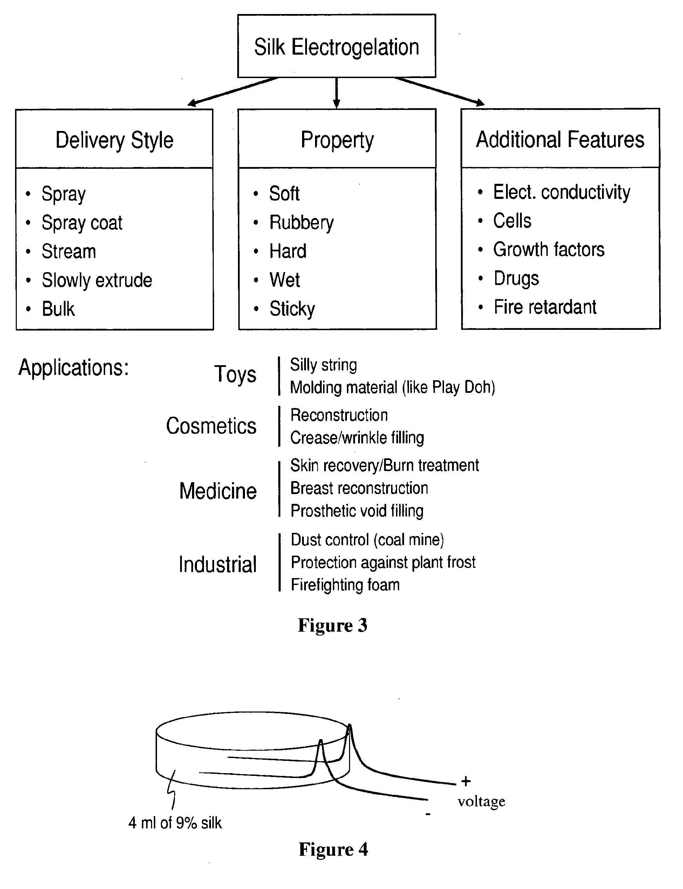

Potential applications are depicted in Figure 3, which describes various delivery styles, achievable gel properties, and additional features that are encompassed in the present invention. Electrogelated silk may be delivered in the form of a spray, stream, slow extrusion, and for creation of gel in bulk. Gel processing and manipulation have shown that soft, rubbery, hard, wet, and sticky consistencies can be achieved. Combinations of these properties are also achievable. For example, a soft, wet, and sticky form of the gelated silk can be created. These styles, properties, and features provide significant opportunity for electrogelation in many potential applications.

For example, SILLY STRING^{®} party string (Just For Kicks, Inc.), is an aerosol can that ejects a liquid that forms into a sticky stream of brightly-colored plastic string. *See e.g*., the How Stuff Works web site. Although mainly marketed to children, the product has found a new application with the U.S. military in Iraq, where soldiers use it to reveal well-disguised trip wires on doorways and in rooms, thereby preventing serious harm to personnel. Santana, *N.J. woman collects Silly String for serious mission in Iraq,* USA TODAY (December 11, 2006). Although the product is very popular, especially with children and during celebrations, it can be harmful when used around an open flame, can cause damage to wallpaper and other materials, and is not biodegradable and may be particularly harmful to marine life. Indeed, some locales such as Los Angeles (where clean-up from Halloween-night party string exceeds $200,000) have banned it. LAMC § 56.02 (2004). A silk-based party string can be also ejected in a flexible stream; but unlike existing party string, the silk gel version is biodegradable and biocompatible, causing less harm to the environment and providing higher safety around children. In fact, the silk gel may also be flavored and ingested with minimal risk.

Another embodiment of the present invention provides for foams or gels for use in firefighting. Various firefighting gels are available commercially. For example, THERMO-GEL^{®} gel concentrate (Thermo Technologies, LLC) transforms into a Class A fire retardant gel when added to water. It can be effective for fighting fires, in aviation applications, and for protecting structures. Another commercially available firefighting gel, AURORAGEL^{™} breakable fire gel (McCoy & Assoc., Inc.) stops and prevents fires by restricting availability of oxygen, isolating the fuel source with a layer of gel, and by lowering the temperature of what is burning. AURORAGEL^{™} fire gel, essentially a thick layer of gelled water, ensures that water stays in contact with surfaces. It can be sprayed on horizontal and vertical surfaces, but also on firefighters for additional protection. This gel can be delivered with a nozzle, contains safe gelling agents, and can be removed with calcium-containing water. Utilizing the electrogelation process described herein, silk-based gels may be utilized in firefighting applications. The ability to quickly gelate silk solution and spray it through a nozzle has been demonstrated. Utilizing chemical modifications the fire retardancy of the silk gel may be improved. The resulting gel may be composed of an all-aqueous system that is biocompatible and biodegradable and has the ability to be sprayed onto vertical and horizontal structural elements, and onto firefighters themselves for additional protection.

An important embodiment of the present invention provides for medical applications such as burn and wound treatment. Burn victims require treatment strategies that involve palliative measures to cool affected tissue and prevent infection to more intensive strategies that involve removal of severely damaged tissue and replacement with suitable material(s). Currently, there are many products on the market that can be used for burn treatment in emergency first-aid scenarios. For example, Water-Jel Technologies (Carlstadt, NJ) produces blankets, dressings, and gels that incorporate a water-based hydrogel that has both bacteriostatic and biodegradable properties. The gel cools the burned area, can protect against infection, and can include lidocaine to relieve pain. BURNFREE Products (Sandy, UT), has a similar line of burn treatment products. Their highly viscous gel remains in place on a burn wound, but can be rinsed off completely for further treatment. The gel contains anti-evaporative, thickening, and preservative additives to ensure functionality under expected usage conditions. Other more sophisticated products derived through tissue engineering research are becoming available on the market. OASIS^{®} wound matrix from Cook Biotech, Inc. (West Lafayette, IN) is a material, derived from porcine sources, that may be used to treat second-degree bums, providing a moist environment that allows the patient's cells to replace damaged tissue.

For moderate skin bums, where significant damage has occurred, autografting can be one treatment strategy. Autografting involves replacement of damaged skin with healthy skin from elsewhere in the body. As an alternative to this approach, a product from Genzyme (Cambridge, MA), known as EPICEL^{®} cultured epidermal autograft, can be used as a permanent skin replacement. By using the patient's skin cells (co-cultured with mouse cells), an allograft can be cultured without fear of immune rejection. The process may take two weeks, producing enough replacement skin to cover an entire body. EPICEL^{®} cultured epidermal autograft is used at major burn centers in the U.S. Other products that are used in similar applications include Integra™ bilayer matrix wound dressing (Integra Lifescience Holdings Corp., Ontario, Canada); ALLODERM^{®} acellular dermal matrix (LifeCell Corp., Branchburg, NJ); ORCEL^{®} bilayered cellular matrix (Forticell Bioscience, Inc., New York, NY); and APLIGRAF^{®} living skin patch (Organogenesis Inc., Canton, MA).

A silk-based hydrogel product may have a significant impact in the treatment of wounds, particularly burn wounds. Because the silk gel is water-based and biodegradable, it can be easily applied or removed without generating waste. Its adhesive qualities and high viscosity allow it to stay in place, cooling the wound and preventing airborne contamination. With the application of tissue engineering approaches and recombinant techniques, cells, drugs, and/or other bioactive molecules may be incorporated into silk gels to protect the wound and stimulate tissue regeneration. For example, if the silk fibroin peptide employed in the electrogelation process does not include a recombinant growth factor such as VEGF or BMP-2, these may be conjugated to the fibroin protein or added to the fibroin solution at some suitable time before electrogelation is undertaken.

Furthermore, because silk gel may be manipulated to effect structural conformation, particularly in triggering β-sheet conformation, silk gel may be particularly useful in bone repair. For example recombinant silk peptides may be electrogelated to silk I conformation peptides in combinations of the silk consensus repeats and added helical domains in a parallel fashion with respect to the folding of the beta-sheets. In this way the dipole moments of the silk I conformation combine in an additive fashion and do not cancel each other out, leading to a net dipole moment of the material, which gives rise to bulk piezoelectric behavior as described above. By varying the content of the silk I domains, the net piezoelectric response can be tailored. Additionally, when producing a bulk material, the protein containing the silk I domains can be blended with unmodified silk proteins to increase the net strength of the system and alter the net piezoelectric properties of the system. The bulk material must be sufficiently anisotropically oriented such that a net dipole is present. By utilizing silk I conformations from the electrogelation process, which themselves self-assemble in a parallel manner, the overall system exhibits increased orientation. For production of fibers one can extrude protein from a narrow orifice to induce molecular alignment. Slow drying of the systems can aid in the most energetically favorable parallel arrangement resulting in a net dipole moment for the system. Initial materials may be tested on a microscopic level by AFM wherein the electrical response of a region is measured as the AFM tip impacts the surface. Measurements on bulk materials can be performed via a number of techniques including load testing while coupled to an electronic load. Electrogelation thus offers the ability to study and harness the relative ratios of silk-helical content in the protein matrixes, to optimize piezoelectric features related to mechanical properties, and identify suitable options in forming bulk systems useful for the bone repair system.

For example, because silk electrogelation may be achieved with hand-held commercial Li-Pol batteries, the present approach may prove extremely useful for first responders such as paramedics or field doctors in the military. Solubilized silk fibroin solution, which has less volume than silk gel, is aqueous and stable at ambient temperature, may be carried in a device equipped with a battery and a switch, such that when the silk gel is needed (e.g., to cover a wound), the medical personnel can expose a portion of the silk solution to the battery-operated field, and within ten minutes have silk gel suitable for use on the patient.

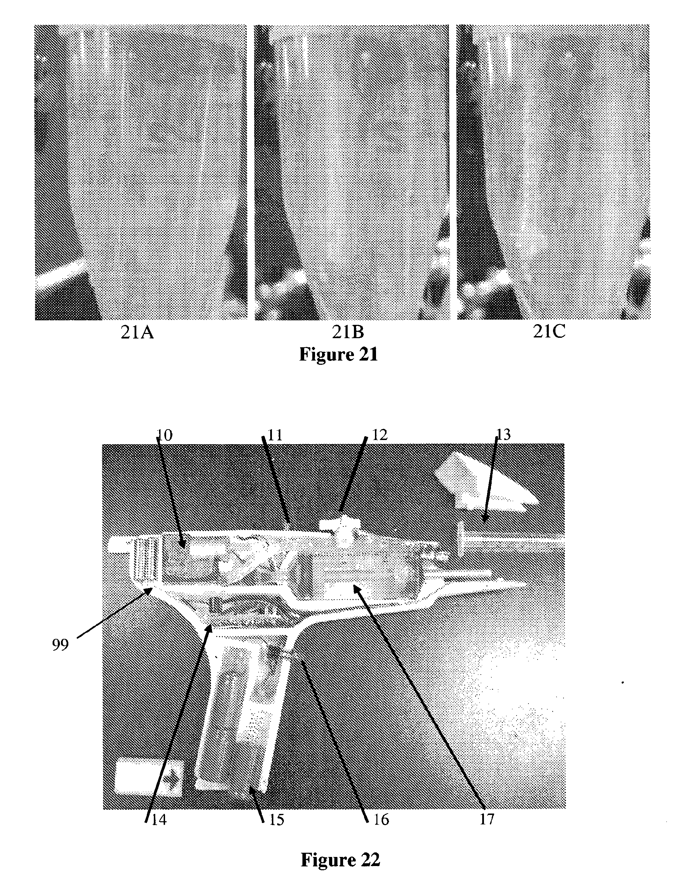

For example, with reference to Figure 22, this is an embodiment of a device and a housing (99) for generating internally electrogelated silk and delivering a steady stream for biomedical-type application, for instance. In use, the operation valve (12) is opened and the silk cartridge (13) containing silk fibroin solution is inserted. At this point, an LED displays a standby signal (e.g., green). The user presses the process button (11), which triggers the timing and logic board (14) to generate an electric field using batteries (15), which may be rechargeable batteries. The LED displays a different color (e.g., red), indicating that processing is underway. The timing and logic board (14) also adjustably monitors time for the gelation processing. When the preset time elapses, the LED changes color (e.g., back to green), and the user then closes the operation valve (12). When the user presses the extrusion button (16), the hydraulic pump (10) and the hydraulic drive (17) engage to eject the silk gel from the tip of the unit.

A device for the delivery of silk gel to a desired location may also use a special silk cartridge device as shown in Figure 23. In this figure, DC voltage is supplied by batteries and creates an electric field in the silk solution (22) via a positive platinum electrode (23) immersed in the silk solution, and a tube-shaped negative electrode that also acts as a valve (21). Also shown is a nozzle attachment (20), which may be removed and exchanged for a nozzle with a larger or smaller aperture to effect the shear forces applied to the silk gel as it is ejected from the nozzle (20). By applying a selected shear force to the electrogelated silk, via the nozzle, the molecular conformation of the meta-stable silk gel may be manipulated from, e.g., silk I to beta-sheet, impacting the solubility and mechanical properties of the extruded silk gel.

Another embodiment of the present invention provides for an the electrogelation of silk fibroin for use as a medical filled material in cosmetic surgery or reconstruction. Current approaches to cosmetic enhancements may use injections of BOTOX^{®} botulinum toxin type A (Allergan Inc., Irvine, CA), to paralyze facial muscles, temporarily eliminating frown lines or wrinkles. Alternatively, COSMODERM^{®} collagen dermal filler and Cosmoplast^{®} collagen dermal filler are "human-based" protein implants, and ZYDERM^{®} and ZYPLAST^{®} collagen implants are bovine-derived products that have been FDA-approved for smoothing facial lines, wrinkles and scars. Other materials, such as hyaluronic acid, can fill thin lips and facial creases. Hydroxylapatite, a mineral-like material found in human bones, can fill deeper creases. Additionally, adipose (fat) tissue can be harvested from the patient's own body and injected in facial locations where fillers build up creases or smooth wrinkles. Certain traumas or diseases that require the removal of tissue in affected areas can leave defects or voids that need filling. For example, a mastectomy results in the creation of large void space in the breast that could be eliminated by suturing a skin fold over where the breast once existed or by refilling the breast with an implant or adipose (fat) tissue harvested from the patient. Due to lack of revascularization, reduction in graft volume can be an issue. Tissue engineering approaches to adipose tissue for breast reconstruction have been pursued. Porous polymer foams, such as from PLGA, have been seeded with preadipocytes to generate adipose tissue formation. These materials are thought to be too stiff, making the patient uncomfortable. Injection of materials, such as alginate and hyaluronic acid hydrogels, has also been investigated. Patrick, 19 Seminars Surgical Oncol. 302-11 (2000).

Electrogelated silk may serve in both cosmetic surgery and for reconstructive surgeries. Silk hydrogels are easily tailored to have the properties desired for the particular application. For example, in breast reconstruction, a soft silk gel injection may provide a higher level of comfort for the patient than reported for implanted PLGA. The demonstrated ability to cell-seed silk-derived gels that can differentiate into viable engineered tissues is attractive for many surgical applications. If a rapid prototyping capability can be developed based on silk electrogelation, potentially 3D images scanned from a patient prior to surgery can be used to generated a 3D tissue engineering scaffold. Applying known tissue engineering strategies, seeded cells may differentiate into adipose tissue, providing both geometry that matches the patient's natural breast geometry and long-term stability.

As noted, electrogelated silk may be modified to allow for encapsulation of at least one agent into silk gels. The agent may be introduced into the silk fibroin solution before, during or immediately after applying voltage to the silk solution to generate an electric field. Some agents may be affected adversely by the voltage or current and hence may not be introduced into the silk fibroin solution until after the application of the electric field. For example, applying electric current may damage or destroy living cells, depending on the magnitude of the applied voltage and current. Hence caution may be applied to the procedures and conditions of encapsulating agents into the electrogelated silk, for example, the agent may be introduced to silk solution immediately after the application of the electric field.

The agent to be encapsulated into electrogelated silk can be any material capable of being encapsulated in the silk gel. For example, the agent may be a therapeutic agent or biological material, such as cells (including stem cells), proteins, peptides, nucleic acids (DNA, RNA, siRNA), nucleic acid analogues, nucleotides, oligonucleotides or sequences, peptide nucleic acids, aptamers, antibodies, hormones, hormone antagonists, growth factors or recombinant growth factors and fragments and variants thereof, cytokines, or enzymes, antibiotics, viruses, antivirals, toxins, prodrugs, chemotherapeutic agents, small molecules, drugs and combinations thereof. Exemplary agent suitable for encapsulation into electrogelated silk includes cells (including stem cells), erythropoietin (EPO), YIGSR peptides, glycosaminoglycans (GAGs), hyaluronic acid (HA), integrins, selectins, and cadherins; analgesics and analgesic combinations; steroids; antibiotics; insulin; interferons α and y; interleukins; adenosine; chemotherapeutic agents (e.g., anticancer agents); tumor necrosis factors α and β; antibodies; cell attachment mediators, such as RGD or integrins, or other naturally derived or genetically engineered proteins, polysaccharides, glycoproteins, cytotoxins, prodrugs, immunogens, or lipoproteins.

One or more agents may be encapsulated into electrogelated silk. For instance, when encapsulating biological material such as cells, it may be desirable to add other materials to promote the growth of the agent, promote the functionality of the agent after it is released from the encapsulation, or increase the agent's ability to survive or retain its efficacy during the encapsulation period. Exemplary materials known to promote cell growth include, but are not limited to, cell growth media, such as Dulbecco's Modified Eagle Medium (DMEM), fetal bovine serum (FBS), non-essential amino acids and antibiotics, and growth and morphogenic factors such as fibroblast growth factor (e.g., FGF 1-9), transforming growth factors (TGFs), vascular endothelial growth factor (VEGF), epidermal growth factor (EGF), platelet derived growth factor (PDGF), insulin-like growth factor (IGF-I and IGF-II), bone morphogenetic growth factors (e.g., BMPs 1-7), bone morphogenetic-like proteins (e.g., GFD-5, GFD-7, and GFD-8), transforming growth factors (e.g., TGF-α, TGF-β I-III), nerve growth factors, and related proteins. Growth factors are known in the art, see, *e.g.,* Rosen & Thies, CELLULAR & MOL. BASIS BONE FORMATION & REPAIR (R.G. Landes Co.). Additional material to be encapsulated in silk gel may include DNA, siRNA, antisense, plasmids, liposomes and related systems for delivery of genetic materials; peptides and proteins to active cellular signaling cascades; peptides and proteins to promote mineralization or related events from cells; adhesion peptides and proteins to improve gel-tissue interfaces; antimicrobial peptides; and proteins and related compounds.

The electrogelated silk encapsulating bioactive agent enables the delivery of active agents in a controlled released manner. Maintaining the bioactive agent in an active form throughout the electrogelated silk preparation process enables it to be active upon release from the silk gel. Controlled release of the active agent permits active agent to be released sustainably over time, with controlled release kinetics. In some instances, the bioactive agent is delivered continuously to the site where treatment is needed, for example, over several weeks. Controlled release over time, for example, over several days or weeks, or longer, permits continuous delivery of the bioactive agent to obtain preferred treatments. The controlled delivery vehicle is advantageous because it protects the bioactive agent from degradation *in vivo* in body fluids and tissue, for example, by proteases.

Controlled release of the active agent from the electrogelated silk may be designed to occur over time, for example, over 12 hours or 24 hours. The time of release may be selected, for example, to occur over a time period of about 12 hours to 24 hours; about 12 hours to 42 hours; or, e.g., about 12 to 72 hours. In another embodiment, release may occur for example on the order of about 1 day to 15 days. The controlled release time may be selected based on the condition treated. For example, longer times may be more effective for wound healing, whereas shorter delivery times may be more useful for some cardiovascular applications.

Controlled release of the active agent from the electrogelated silk *in vivo* may occur, for example, in the amount of about 1 ng to 1 mg/day. In other embodiments, the controlled release may occur in the amount of about 50 ng to 500 ng/day, or, in another embodiment, in the amount of about 100 ng/day. Delivery systems comprising therapeutic agent and a carrier may be formulated that include, for example, 10 ng to 1 mg therapeutic agent, or about 1 µg to 500 µg, or, for example, about 10 µg to 100 µg, depending on the therapeutic application.

A pharmaceutical formulation may be prepared that contains the electrogelated silk encapsulating bioactive agents. The bioactive agents may be one or more active agents described above. The formulation can be administered to a patient in need of the particular bioactive agent that has been encapsulated in the electrogelated silk.

The pharmaceutical formulation may also contain common components found in other pharmaceutical formulations, such as known excipients. Exemplary excipients include diluents, solvents, buffers, or other liquid vehicle, solubilizers, dispersing or suspending agents, isotonic agents, viscosity controlling agents, binders, lubricants, surfactants, preservatives, stabilizers and the like, as suited to particular dosage form desired. The formulations may also include bulking agents, chelating agents, and antioxidants. Where parenteral formulations are used, the formulation may additionally or alternately include sugars, amino acids, or electrolytes.

Some examples of materials which can serve as pharmaceutically acceptable carriers include, but are not limited to, sugars such as lactose, glucose and sucrose; starches such as corn starch and potato starch; cellulose and its derivatives such as sodium carboxymethyl cellulose, ethyl cellulose and cellulose acetate; powdered tragacanth; malt; gelatine; talc; oils such as peanut oil, cottonseed oil; safflower oil, sesame oil; olive oil; corn oil and soybean oil; esters such as ethyl oleate and ethyl laurate; agar; non-toxic compatible lubricants such as sodium lauryl sulfate and magnesium stearate; polyols, for example, of a molecular weight less than about 70,000 kD, such as trehalose, mannitol, and polyethylene glycol. See for example, U.S. Patent No. 5,589,167, the disclosure of which is incorporated by reference herein. Exemplary surfactants include nonionic surfactants, such as Tween surfactants, polysorbates, such as polysorbate 20 or 80, etc., and the poloxamers, such as poloxamer 184 or 188, Pluronic polyols, and other ethylene/polypropylene block polymers, etc. Suitable buffers include Tris, citrate, succinate, acetate, or histidine buffers. Suitable preservatives include phenol, benzyl alcohol, metacresol, methyl paraben, propyl paraben, benzalconium chloride, and benzethonium chloride. Other additives include carboxymethylcellulose, dextran, and gelatin. Suitable stabilizing agents include heparin, pentosan polysulfate and other heparinoids, and divalent cations such as magnesium and zinc. Coloring agents, releasing agents, coating agents, sweetening, flavoring and perfuming agents, preservatives and antioxidants can also be present in the composition, according to the judgment of the formulator.

The pharmaceutical formulation may be administered by a variety of routes known in the art including topical, oral, ocular, nasal, transdermal or parenteral (including intravenous, intraperitoneal, intramuscular and subcutaneous injection as well as intranasal or inhalation administration) and implantation. The delivery may be systemic, regional, or local. Additionally, the delivery may be intrathecal, e.g., for CNS delivery.

The pharmaceutical formulations may contain common components found in other pharmaceutical formulations, such as known excipients. Exemplary excipients include diluents, solvents, buffers, or other liquid vehicle, solubilizers, dispersing or suspending agents, isotonic agents, viscosity controlling agents, binders, lubricants, surfactants, preservatives, stabilizers and the like, as suited to particular dosage form desired. The formulations may also include bulking agents, chelating agents, and antioxidants. Where parenteral formulations are used, the formulation may additionally or alternately include sugars, amino acids, or electrolytes.

Some examples of materials which can serve as pharmaceutically acceptable carriers include, but are not limited to, sugars such as lactose, glucose and sucrose; starches such as corn starch and potato starch; cellulose and its derivatives such as sodium carboxymethyl cellulose, ethyl cellulose and cellulose acetate; powdered tragacanth; malt; gelatine; talc; oils such as peanut oil, cottonseed oil; safflower oil, sesame oil; olive oil; corn oil and soybean oil; esters such as ethyl oleate and ethyl laurate; agar; non-toxic compatible lubricants such as sodium lauryl sulfate and magnesium stearate; polyols, for example, of a molecular weight less than about 70,000 kD, such as trehalose, mannitol, and polyethylene glycol. *See e.g.,* U.S. Patent No. 5,589,167. Exemplary surfactants include nonionic surfactants, such as Tween surfactants, polysorbates, such as polysorbate 20 or 80, etc., and the poloxamers, such as poloxamer 184
or 188, Pluronic polyols, and other ethylene/polypropylene block polymers, etc. Suitable buffers include Tris, citrate, succinate, acetate, or histidine buffers. Suitable preservatives include phenol, benzyl alcohol, metacresol, methyl paraben, propyl paraben, benzalconium chloride, and benzethonium chloride. Other additives include carboxymethylcellulose, dextran, and gelatin. Suitable stabilizing agents include heparin, pentosan polysulfate and other heparinoids, and divalent cations such as magnesium and zinc. Coloring agents, releasing agents, coating agents, sweetening, flavoring and perfuming agents, preservatives and antioxidants can also be present in the composition, according to the judgment of the formulator.

The pharmaceutical formulations containing the electrogelated silk encapsulating therapeutic agent can be administered in a controlled-release manner so that portions of the therapeutic agent are released in the patient over a period of time. The therapeutic agent may release quickly or slowly. For instance, the pharmaceutical formulation can be administered so that less than about 5% of the therapeutic agent is released in the patient from the electrogelated silk over a period of one month. Alternatively, a larger portion of the therapeutic agent may be released initially, with a smaller portion retained in the silk gel and released later. For example, the pharmaceutical formulation can be administered so that at least 5% of the therapeutic agent remains in the silk 10 days after administration.

The electrogelated silk may encapsulate various other active agents useful in a variety of fields. For instance, the active agent may be an enzyme or an enzyme-based electrode. The enzyme or enzyme-based electrode may be used in the field of tissue engineering, biosensors, the food industry, environmental control, or biomedical applications. The system can also be used as a reservoir for a variety of needs, such as in the food industry to harbor vitamins, nutrients, antioxidants and other additives; in the environmental field to harbor microorganisms for remediation or water treatments; in materials as additives to serve as a source of in situ detection and repair components, such as for nondestructive evaluation of material failures and self-repairs of the materials; and for biodetection schemes to help stabilize cells, molecules and related systems.

As noted herein, silk fibroin gels are edible and may be flavored. Hence, a flavored silk gel formulation of vitamins, nutraceuticals, or other pharmaceuticals may be produced for pediatric use. The gels may be preformed and packaged, or prepared as a solution in a suitable dispenser that will create the gel at the office or home under adult supervision.

Another embodiment of the present invention provides for electrogelated silk for use as biodegradable and biocompatible coatings on tissues, regenerated tissues, medical devices, and medical implants. Biodegradable and biocompatible implant coatings are of clinical interest due to their ability of continuously eluting desired drugs (e.g. anti inflammatory medication or growth factors) to the surrounding tissue of the implant. Additional drug delivery methods such as intravenous delivery thus can be avoided. By embedding drug in the implant or device coating, the drug is concentrated at the target site in a higher dosage and is less spread over the whole organism.

Current biodegradable biocompatible implant coatings such as PLA or PGA films involve elevated processing temperatures or harsh conditions that can damage the activity of drugs to be incorporated. In addition, due to the limitation of the conventional coating technique and coating materials, the conventional degradable implant coatings are only applicable to simple implant forms, and are not suitable for the coating of complex implant geometries such as implants having internal configurations or undercuts. The modern advanced biomechanical designs, however, often lead to complex implant forms that are used in biomedical field such as dental implantology, traumatology, orthopedics and cardio vascular applications. Hence a novel type of biodegradable biocompatible implant coating material and techniques are needed for complex implant designs such as spine cages, coronary stents, dental implants or hip and knee prostheses.

Electrogelation may prove superior to other medical device coating technique since electrogelation can be applied using a wide range of electrode materials and geometries, from platinum wire electrodes to mating metal plates to tissues (in vitro or in vivo) that can be coated with silk. Silk e-gel (silk e-gel, defined herein, refers to the electrogelated silk gel) in accordance with the present invention hence may be used as thin film coatings or bulk coatings on tissues or a variety of medical devices and medical implants.

The silk e-gel coating technique herein employs the silk electrogelation process described in the embodiments of the present invention. In a simple example for silk e-gel coating process, aqueous silk fibroin solution can be locally turned in a gel-like state by means of applying electric energy. The medical device such as an implant is used in effect as the positive electric pole (e.g., the implant or device is attached to the positive electric pole of the circuit) and is immersed into the aqueous silk solution until all surfaces of the device desired to be coated are in contact with the solution. When applying appropriate voltage and current (e.g., U=25V, I=10mA), the aqueous silk solution surrounding the positive electric pole (the device) almost immediately turn into a gel like structure (so called e-gel effect) and the gel-like structure grows from the solution-contacting surface (either exterior surface or interior surface of a complex geometry) of the device to the surrounding silk solution until the electronic circuit is opened. The e-gel effect may be applied to any substrate (e.g., tissues or medical devices) inserted into the silk solution and can generate a homogenous biodegradable and biocompatible silk e-gel coating on the substrate.

The silk e-gel coating may be either in a form of bulk gel coatings or as a thin film coating. For example, the electrogelation process described above can generate a homogenous bulk silk e-gel coating that is growing on the surface of the substrate. Optionally, the silk e-gel coated substrate may be further treated with a dehydrating treatment method to achieve a stable beta-sheet conformation. Dehydrating treatment may refer to drying the silk e-gel coated substrate in the air or exposing the silk e-gel coated substrate to a flow of dehydrating gas such as nitrogen gas or a dehydrating solvent such as methanol. For example, drying the silk e-gel coating can induce the evaporation of water and convert the silk e-gel coating to a mechanically stable silk thin film on the substrate.

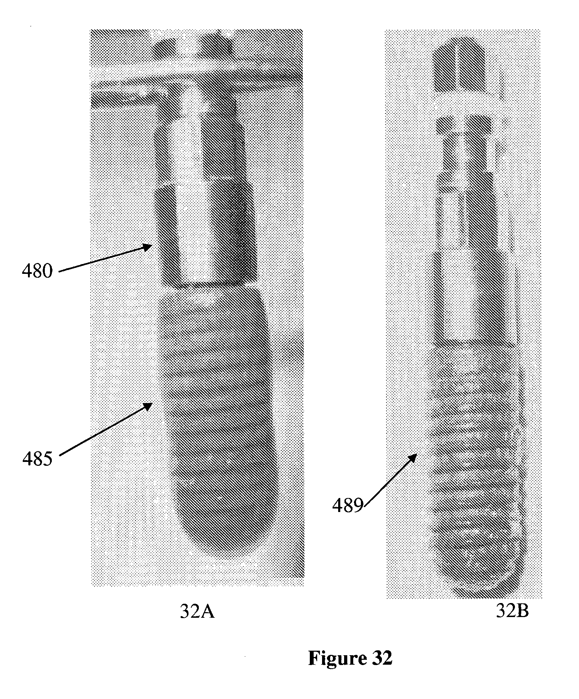

Figure 32A illustrates a Straumann® dental implant (BL RC, ø 4.1mm, 10mm SLActive®, Straumann, Basel, Switzerland) coated with silk e-gel via the described direct electrogelation technique. The dental implant 480 is used as the positive electric pole and is inserted into about 4.5% silk solution. A voltage potential of 25V was applied on the dental implant 480 with a current of 10mA for 10 seconds. A homogenous silk e-gel 485 is then formed on the dental implant 480. The silk e-gel coated dental implant 480 shown as in Figure 32A may be dried for 2 hours at room temperature to evaporate water in the e-gel.
The silk e-gel then shrinks and forms a mechanically stable silk thin film 489, as shown in Figure 32B.

The substrates for coating of the electrogelated silk may include any number of tissues or regenerated tissue materials or medical devices and implants as well as any instrument, apparatus, appliance, material or other article that are used alone or in combination for diagnostic and/or therapeutic and/or surgical purposes and their proper application. For example, silk e-gel coating containing cell growth factor may be applied to skin tissue in vivo to promote burn wound healing. Exemplary medical devices and implants include devices and implants in dental applications such as dental filling materials, dental prosthetic devices (e.g., crowns, bridges, removable prostheses), dental implants (e.g., osseointegrated implant), and orthodontic appliances (e.g., dental braces or retainer); devices and implants in orthopaedic applications such as bone filling materials, arthroplasty devices (e.g., hip and knee prostheses), orthopedic implants (e.g., hip, knee, shoulder and elbow prosthesis, bone cements, prosthetic joint components), maxillofacial implants and devices (e.g., skull, face, and jaw prostheses), spinal devices or implants (e.g., spinal cord, spinal column, spine cage) and osteosynthesis devices and instrumentation for fixation of bones and/or joint components (e.g., nails, wires, pin, plates, rods, wires, screws or other fixators); devices and implants in traumatology applications such as wound healing devices or implants (skin, bone, and vascular wound grafts and patches, sutures, vascular wound repair devices, hemostatic dressings); cardiovascular devices and implants such as blood vessel prosthesis, stents, catheters, vascular grafts, heart valves and pacemakers; coagulation devices such as argon enhanced coagulation devices with coated probes; implantable artificial organs (e.g. artificial heart, liver, kidney, lungs, brain pacemaker and the like); or other small implantation materials such as occluders, filters, screws, bolts, nuts, pins, plates, gears, sleeve bearings, washers, anchors (for suture and/or tendon anchoring), plugs, nails and the like. Additionally, medical instruments such as scalpels, forceps, scoops, currettes, steotomes, chisels, gouges, strippers and the like maybe coated with a silk e-gel and active agent such as antibiotic or anticoagulation drugs may be embedded in the silk e-gel coating to further effect patient outcomes. Likewise, veterinary implants, devices, and instruments may also include a silk fibroin gel in accordance with the present invention.

The substrate for coating of the electrogelated silk can be any materials that are suitable to be coated with silk gel through the electrogelation process. For example, the substrate may be electrically conductive materials such as metals, alloys, electrical conductive polymers or ceramics, or combination thereof. Exemplary metals or alloys for producing the devices and implants include, palladium, titanium, gold, platinum, silver, niobium, stainless steel nickel, tin, copper, tantalum or alloys thereof, cobalt-chromium alloys, or Vitallium (CoCrMo alloy). Exemplary polymers for producing the devices and implants include silicones (breast prostheses, pacemaker leads), polyurethanes (pacemaker components), polymethacrylates (dental prosthese, bone cements), poly(ethylene terephthalate) (vascular grafts, heart valve sewing rings, sutures), polypropylene (sutures), polyethylene (prosthetic joint components), polytetrafluoroethylene (vascular prostheses), polyamides (sutures) and polylactides and poly(glycolic acids) (bioresorbables). Exemplary ceramic. materials for producing the devices and implants include metal oxides (alumina, zirconia) that are generally used in joint replacements and dental prostheses; Other materials such as materials based on calcium phosphate (bone fillers) and materials based on pyrolytic carbon (heart valves) may be also be used as the substrate for silk e-gel coating.

Optionally, one or more active agents may be embedded in a silk e-gel coating to be used as a functional coating. Alternatively, active agents can be loaded onto a pre-formed silk e-gel coating. The pre-formed silk e-gel coating may or may not contain an active agent, and the active agent in the pre-formed silk e-gel may or may not be the same as the active agent to be loaded onto the pre-formed silk e-gel coating. Alternatively, an active agent may be coated to the substrate as the first layer coating before the electrogelation process and a silk e-gel layer may be subsequently coated on the active agent-coated substrate. The active agent then will not be released until the silk e-gel is degraded. The active agents may be present as a liquid, a finely divided solid such as drug particles or any other appropriate physical form.

The active agent may be a therapeutic agent or a biological material such as the agents described above. By coating tissues or medical devices with the silk fibroin get of the present invention, the tissue or device may provide further benefits than a similarly uncoated tissue or device. For example, wounded skin tissue may be coated with silk e-gel coating containing at least one cell growth factor (e.g., epidermal growth factor) to promote burn wound healing. Such silk e-gel coating process of wounded silk tissue may be performed *in vivo* to stimulate cells to re-epithelialize the wound. Alternatively, the silk e-gel coating process may also be performed *in vitro* on tissues or regenerated tissues for subsequent implantation. In another example, an implant may be coated with a silk e-gel containing an antibiotic or antiinflammatory drug. Upon implantation, the gel may dissolve, and the antibiotic or antiinflammatory drugs may be released as near to the incision or implant area as possible to achieve the lowest surgical site infection rates. Additionally, a drug embedding silk e-gel coated medical device or implant may reduce the need to frequently administer a drug to maintain adequate therapeutic concentrations.

Electrogelation has been shown herein to be reversible. In simple terms, electrogelation involves the conversion of a solubilized random-coil silk solution into a gel with significant silk I content by the application of an electric field via DC voltage. When the DC voltage is reversed (reversal of the electrode polarity), the silk gel tends to convert back to the random coil conformation. Indeed, the reversing process may be driven to near completion, and the electrogelation/reversing process can be repeated many times. Importantly, the nature of the silk material changes dramatically when alternating from the gel state to the liquid silk solution state: The gel has a very sticky, thick, mucus-like consistency. The liquid silk solution is not sticky at all and has a very low viscosity compared to the gel. The electrogelated silk, therefore, can be used as an active silk muco-adhesive, analogous to the slime secretion that snails and slugs utilize for motion and for securing themselves to various surfaces.

Other materials exhibit the ability to change stiffness or consistency based on the addition or subtraction of external energy or fields. For example, a class of fluids, commonly known as "smart fluids", are capable of changing properties in the presence of an electric or magnetic field. Typically, the fluid viscosity is modified with the presence and/or strength of the field applied. Additionally, magneto-rheological fluids contain a suspension of magnetic particles that align in the presence of a magnetic field. The fluid viscosity is effectively increased due to the alignment. Electro-rheological fluids consist of non-conducting particles that are suspended in an insulating fluid. The fluid viscosity can change dramatically in the present of an electric field. Further, thermoresponsive polymers can display material property changes with temperature change, such as solubility state and size.

Another aspect of the present invention relates to muco-adhesives. Typically polymers, these materials can be prepared as hydrogels, films, microspheres, sponges, tablets, or microtablets. Because of their potential for excellent adhesion to multiple surfaces, including wet surfaces, one application area has been in controlled and local drug delivery. MucoAD™ (non-toxic, non-irritating, liquid muco-adhesive carrier, Strategic Pharmaceuticals Co., Washington, DC), is based on the hypromellose [hydroxypropyl methylcellulose (HPMC)] polymer. This product apparently has a high affinity to the mucosa and prolongs time of contact between a drug and mucosal tissue. It has potential for intranasal, ophthalmic (e.g., help relieve dry eye), vaginal, and rectal applications.

Further, a biologically-inspired robot that uses the friction of muco-adhesives to perform colonoscopies is being developed. Using balloon-like actuators to drive snail-like movement within the colon, the biological robot design relies on the use of materials at the robot-colon interface that provide the correct amount of friction and/or adhesion. This application employs a solid muco-adhesive film known as CARBOPOL^{®} crosslinked acrylic acid-based polymer (NOVEON^{®} Consumer Specialties, Wickliffe, OH), a high molecular weight cross-linked polymer of acrylic acid. This material can attach to mucus with physical bonds that form quickly. CARBOPOL^{®} polymer may be used in tape form and successfully adhere to a pig colon, although shear damage limits the tape to single use application. Thus, every step of the robot required that new tape be dispensed (Dodou et al., Proc. 12th Int'l Conf Adv. Robotics 352-59 (Seattle, WA, 2005). Water could be introduced to overcome the force of fiction, allowing the tape to be disengaged. Dadou et al., 15(5) Minimally Invasive Therapy 286-95 (2006).

Although controlled drug delivery systems are available and significant progress has been made, there is still a significant need for systems that administer drugs in a manner that precisely matches physiological needs. Moreover, there is a need for tools to deliver peptide and protein drugs. Hydrogels have been used in drug delivery systems, protecting the drug and allowing controlled drug release through changes in gel structure driven by external stimuli. Stimuli that affect hydrogels include temperature, electric fields, solvent composition, light, pressure, sound, magnetic fields, pH, and ion introduction. Temperature-sensitive polymers, such as poly(N-iso-propylacrylamide) (PNIPAAm) and Poly(N,N-diethylacrylamide) (PDEAAm) are used extensively. Poly(propylene oxide) PPO and poly(ethylene oxide) PEO block copolymers have been used in controlled drug delivery based on sol-gel conversion at body temperature (e.g., PLURONICS^{®} block copolymers, BASF).

Another class of stimulus-sensitive hydrogels are electro-sensitive hydrogels. Such hydrogels, which are also typically pH-sensitive, are usually made of polyelectrolytes and undergo shrinking or swelling in the presence of an applied electric field. Such materials have been applied in drug delivery. For example, hydrogels of poly(2-acrylamide-2-methylpropane sulfonic acid-co-n-butylmethacrylate) were used to release edrophonium chloride and hydrocortisone in a pulsatile manner using an electric current in distilled-deionized water. Electric fields have also been used to control erosion of hydrogels made of poly(ehtyloxazoline)-PMA complex in saline solution. Typically, these hydrogels may also be actuated with an external electrical field. One of the limitations is difficulty in achieving the response in electrolytes; in other words, under physiological conditions. Oui & Park, 53 Adv. Drug Deliv. Rev. 321-39 (2001). Chemically crosslinked hyaluronic acid (HA) hydrogels have also been shown to be electro-sensitive. Using an electric field, pulsed release of negatively charged macromolecules can be achieved. Tomer et. al., 33 J. Contr. Release 405-13 (1995)).

The versatility of silk and availability of different forms, conformations, and processing techniques opens up a wealth of potential application areas. Figure 39 provides a brief overview of the processing strategies, predicted properties, and potential application areas. Note that processing techniques may be performed in sequence. The process labeled "Egel" is the electrogelation process described herein. Vibration can be applied in the form of high-intensity energy generated by an ultrasonicator or lower-intensity energy generated by an electric toothbrush-style actuator. Shearing force can be provided by straining a material through hands-on manipulation or by extruding it through an orifice (flow-induced shear). An oven may be used to effect fast drying, although a room-temperature process provides slow drying. It should be noted that this is not meant to be an exhaustive list, but to provide a context for the electrogelation process. Other processing techniques, such as pH modification, methanol treatment, and water annealing are also used to achieve desired silk outcomes.

Based on results of experiments and experience working with silk and silk-based materials, it is clear there are many potential applications of active silk muco-adhesives described herein, including robotic applications (non-biomedical) and biomedical applications.

There are many applications in the area of robotics for a material that can transition from very sticky to non-sticky. Wall- and ceiling-climbing robots are being pursued for applications that range from military and police surveillance to explosives placement, as well as for green chemistry adhesive and related applications. Active silk muco-adhesives would have a direct application in providing a controlled adhesion to walls and ceilings. The properties of the adhesive gel created through electrogelation seem to mimic the slime that land snails (e.g., *Helix lucorum*) produce. These snails extrude the slime out of their underside, creating a slimy path on which they crawl. In addition, these animals extrude a slightly more adhesive version of the slime to anchor themselves to leaves, rocks, or other surfaces. In the snail, locomotion is achieved through a wavelike response in its muscular foot (underside). The back section of their foot pushes forward, sliding in the slime. When stopped, the foot sticks in place. The section of foot in front of the stuck section then slides forward in the slime. In this way, the snail or slug slowly moves forward along the slime trail.

This method of locomotion may be easily utilized with the silk muco-adhesive, because the silk muco-adhesives can be utilized in two ways: providing a sticky slime that a robot can crawl along and as an adhesive anchoring system. This application does not need to take advantage of reverse gelation. In other words, the adhesive does not need to be active. A second form of locomotion can be achieved with the use of active adhesion. For example, individual robot 'feet' can contain small dispensers and electrogelation coils. When the robot needs to stick to a wall or ceiling, silk solution can be extruded through the appropriate dispenser and converted to gel through electrogelation. When the foot needs to be removed from the wall, reverse electrogelation converts the gel back to a liquid, eliminating the adhesion.

In surveillance applications, it can be important to stay in place for a long period of time. It is also important that on-board energy is not wasted in maintaining a position for this period of time. Silk muco-adhesives can be used to anchor a robot or other devices to various surfaces for an extended period. Electrogelated silk gel can adhere an object temporarily if reverse electrogelation or an applied force causes disruption of adhesion. It can also be used for long-term adhesion. It has been shown that an object can adhere to a painted wall surface for days and potentially weeks without expending energy. The limiting factor is potentially the length of time for the silk material to degrade. Another application area in robotics is in terms of payloads. The mission of a robot might be to deliver a camera, antenna, or other device for surveillance. Silk muco-adhesive can be used to anchor any such device to a wall for short-term or long-term surveillance. One strategy can be to have the robot extrude a steady stream of electrogelated silk with a fine wire embedded. The wire would adhere to the wall because of the muco-adhesive and act as an antenna.

Active silk muco-adhesives open up a range of missions for robots that heretofore have not been fully explored. Small-sized, snail-like robots can be constructed that fundamentally are based on the slime-dependent motion of snails. Like snails, silk muco-adhesives can allow a robotic snail to adhere to a variety of surfaces, ranging from smooth to rough. The types of surfaces can include glass, acrylic, wood, painted walls, cement, rocks, dirty and dusty surfaces, to name a few. Such robots could be used to: inspect the inside or outside of pipes, even with the presence of fluids; be used in forested areas to detect the intensity and presence of fires; help in rescue situations, such as collapsed houses or mines; be utilized on building surfaces or various natural surfaces, such as trees or rocks, to take data during severe weather, like tornados; be deployed in animal preserve areas to track endangered animals; or attach to animals in a temporary manner to allow study. Police or other rescue services could throw miniature versions of the robot against walls or road signs to provide a temporary method of gaining information, such as wireless video feeds.

Using this approach, a silk muco-adhesive was used to attach a surveillance platform to a wall. The platform contained a wireless video camera that streamed live images to a laptop, while a remote controller was used to move the camera on a motorized pivot. The controller was also used to provide controlled reverse gelation, which caused the platform to be gravity-fed down a surface. Because of the unique ability of silk muco-adhesive to adhere to underwater surfaces, a robot using a silk muco-adhesive in accordance with the present invention can perform many underwater missions: inspection of oil platforms and other underwater structures; covert attachment to boats and subs for surveillance and communication interception; and surveying of riverbeds and water floors. Perhaps swimming robots could use homing devices, such as sound or temperature sensors to find a ship, then attach via electrogelation; repair or reconnaissance missions could then be carried. To accommodate longer-duration missions, silk solution maintained at a high pH can be carried in on-board storage, the higher pH extending the longer storage-life of the silk.

Additionally, there is a wide range of biomedical application areas for active silk muco-adhesive. Given the tremendous potential to create a snail-like robot, a biomedical version can be created for medical diagnostics or treatment. For instance, a miniature version can enter the body either through swallowing or insertion through a small incision. The biomedical robot can move in, on, or around organs through adhesive locomotion. The ability to adhere to wet surfaces allows the robot to position itself near damage or diseased locations. Sensors or a camera can be used to relay critical information to medical personnel. Silk gel, stored inside the robot, can be deposited at a damage site, providing an initial temporary repair. The gel can contain drugs, growth factors, antibiotics, or other bioactive ingredients to encourage healing. In conjunction with other functionality, such as the application of vibration or the use of therapeutic ultrasound, the gel patch (in silk I conformation) can be manipulated into a beta-sheet conformation. In this form, the patch is considerably stronger and water insoluble. This allows the patch to have longer-term duration. The material can also be used on burns, wounds, or other locations on the outside of a body. Like for the internal applications, bioactive ingredients can be incorporated for improved healing, and the material is soothing and assists in protecting against infection and contamination. In another biomedical application, silk muco-adhesives can also be used to anchor a pill or other drug-release product to mucosa layers of various tissues. This provides the ability for sustained drug delivery.

Silk muco-adhesive can be useful in robotic design. Many key components in a biomedical robot that explores the inside of a human can take advantage of such materials. Electrogelated silk may also be important as an actuator. When silk solution is electrogelated, there is a stiffness change and also possibly a volume change. These characteristics can be exploited to make an actuator. In one embodiment, silk solution and gel can be used to adjust the angle of a plate. When the plate is used to support a miniature camera, the process can create motion without using a traditional mechanism. Another actuator design consists of a flexible tube or bladder-supported tube. When the silk solution changes volume during electrogelation, the tube can be actuated like a wagging finger. In all of the biomedical applications, the use of silk materials allows slow degradation over time, obviating the need for removing the device from the body.

Further, the systems above can be used to lay down fiber optic cable inside the body in the form of silk fiber optics. Because, as reported previously, silk fibers can serve as light guides, this embodiment offers options for *in vivo* imaging that provides new windows into healing, treating wounds, cancers or vessel deposits, and many related needs.

Aside from the robotic and related fields outlined above, these systems are useful in consumer related products such as toys, science projects, and new forms of artwork among others. Further, the option to functionalize the carrier to deposit, secrete or lay down new materials suggests options in remote construction, sealing actions, hazardous sites, boat hulls, and many other related applications on land, at sea, and in the air.

### EXAMPLES

### Example 1. Parallel electrode gelation

Stock aqueous solutions of solubilized silk fibroin as were prepared as described previously. Sofia et al., 54 J. Biomed. Mater. Res. A 139-48 (2001). See U.S. Patent Application Ser. No. 11/247,358, WO/2005/012606, and WO/2008/127401. Briefly, cocoons of *B*. *mori* were boiled for 20 min in an aqueous solution of 0.02 M sodium carbonate, and then rinsed thoroughly with pure water. After drying, the extracted silk fibroin was dissolved in a 9.3 M LiBr solution at 60 °C for 4 hr, yielding a 20% (w/v) solution. The resulting solution was dialyzed against distilled water using Slide-A-Lyzer® 3.5K MWCO dialysis cassettes (Pierce, Rockford, IL) for three days to remove the residual salt. The solution was optically clear after dialysis and was centrifuged twice at 10,000 rpm for 20 min to remove silk aggregates as well as debris from original cocoons. The final concentration of silk fibroin aqueous solution was approximately 8% (w/v). This concentration was determined by drying the solution with a known volume and weighing the residual solid. The 8% silk fibroin solution was stored at 4°C and diluted with ultrapure water before use. The silk fibroin solution may be diluted or concentrated to a higher concentration. To obtain a silk fibroin solution with a higher concentration, the silk fibroin solution with a lower concentration may be dialyzed against a hygroscopic polymer, for example, PEG, a polyethylene oxide, amylose or sericin. For example, an 8% silk fibroin solution may be dialyzed against 10% (w/v) PEG (10,000 g/mol) solution.

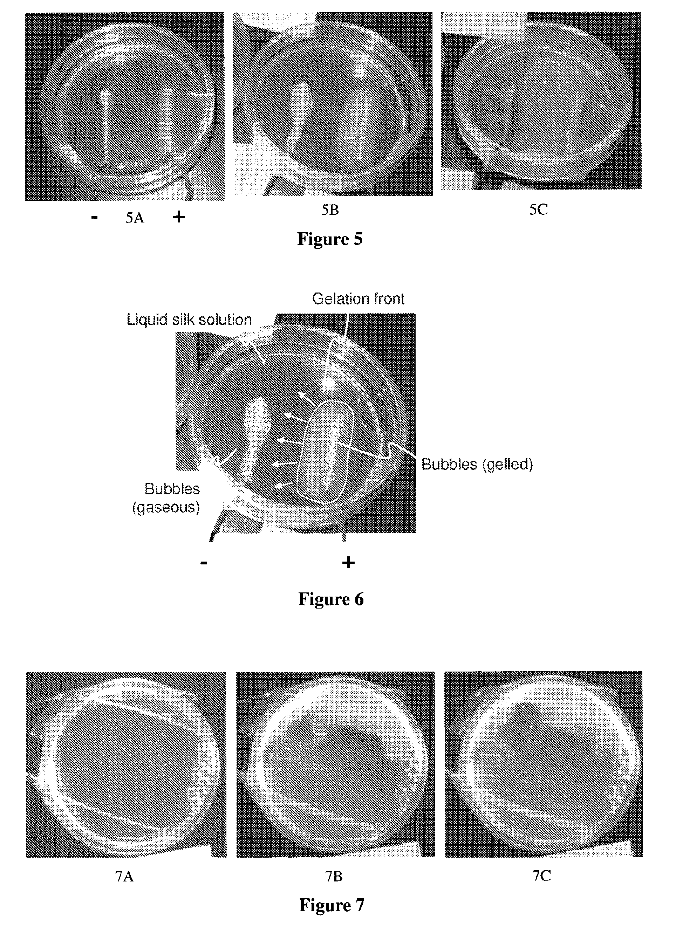

Two parallel wires (electrodes) were placed along the bottom of a 70 mm diameter plastic culture dish that contained 4 ml of ∼9% silk fibroin solution, as shown in Figure 4. The liquid level was slightly above the top of the electrodes and the electrodes were approximately 40 mm apart. A voltage of 22.2 VDC was applied using a high-current Li-Pol battery. Figures 5A, 5B, and 5C shows photos of the initial experiment at time points of 0 min, 20 min, and 275 min, respectively. As shown in Figure 5A and Figure 6, almost immediately upon applying the potential, gelation began at the positive electrode (+) and bubbles formed at the positive (+) and negative (-) electrodes. As shown in Figure 5B, over the first 30 min of electrogelation, the gelation process continued, with steady growth of a "gel front" that emanated from the positive electrode (+) in the general direction of the negative electrode (-).

On closer inspection, radial patterns could be seen within the gelation zone, emanating from the positive electrode and pointing in the general direction of the negative. After about 30 min, gelation continued, but at a slower rate. The bubbles that formed on the positive electrode (+), likely oxygen gas, would become permanent features within the gel, as the solid formed around them. The barrier formed by the permanent bubbles on this electrode may play a key role in the decrease in the rate of gel front growth. Bubbles on the negative electrode (-), likely hydrogen gas, formed constantly, but also dissipated somewhat as their gaseous contents reached the silk solution surface. In all cases, the rate of bubble formation was greater than the rate of dissipation. The consistency of the gelated silk was highly viscous (soft) and very tacky; similar to the consistency of thick mucus. After the culture dish was refrigerated overnight, the gelated silk was quite stiff.

### Example 2. Parallel electrode gelation

The same parallel electrode experimental setup of Example 1 was used with ∼9% silk solution, with the exception that a higher voltage potential of 74.9 VDC was applied across the electrodes to generate the electric field. This tested whether higher voltage accelerates the gelation process and possibly results in more complete gelation. Figures 7A, 7B, and 7C show experimental results at time points of 0 min, 15 min, and 30 min, respectively. As described earlier, a similar gelation process was witnessed. For example, radial patterns in the gelation front are clearly seen in Figure 7B. With the higher voltage, the gelation front expanded at a higher rate, but it also appeared that the electrogelated silk was not as dense as witnessed using a lower voltage. Bubbles on the negative electrode formed at a much higher rate at this voltage, as evidence of an accelerated underlying reaction.

### Example 3. Extrusion test characterizes texture of electrogelated silk fibroin

The ability to rapidly electrogelate a controlled volume of silk solution provides numerous practical applications. A gelated portion of silk generated after 5 hours of electrogelation was extracted and poured into a 10 ml syringe. Using a 1 mm diameter syringe needle attached, the gelated silk was extruded slowly. The silk material ejected from needle was much stiffer than when it was extracted from the dish. This effect can be caused by flow-induced shear as the silk is forced through the small diameter needle. When the electrogelated silk holds a meta-stable silk I conformation, extrusion through a needle causes the additional alignment necessary to convert the microstructure to β-sheet. Many qualitative observations were made from this initial extruded gelation sample. For example, the material was sticky, fairly stiff, quite elastic, and cool to the touch. In fact, the entire gelation process, from electrogelation to extrusion, appears to maintain silk temperature around room temperature. As the silk was extruded through the needle, the resulting extruded gel was ejected as a coherent stream. Upon extruding, the resulting material is translucent. After about 5 min when exposed to air, the color changed to an opaque white.

### Example 4. Magnetic fields

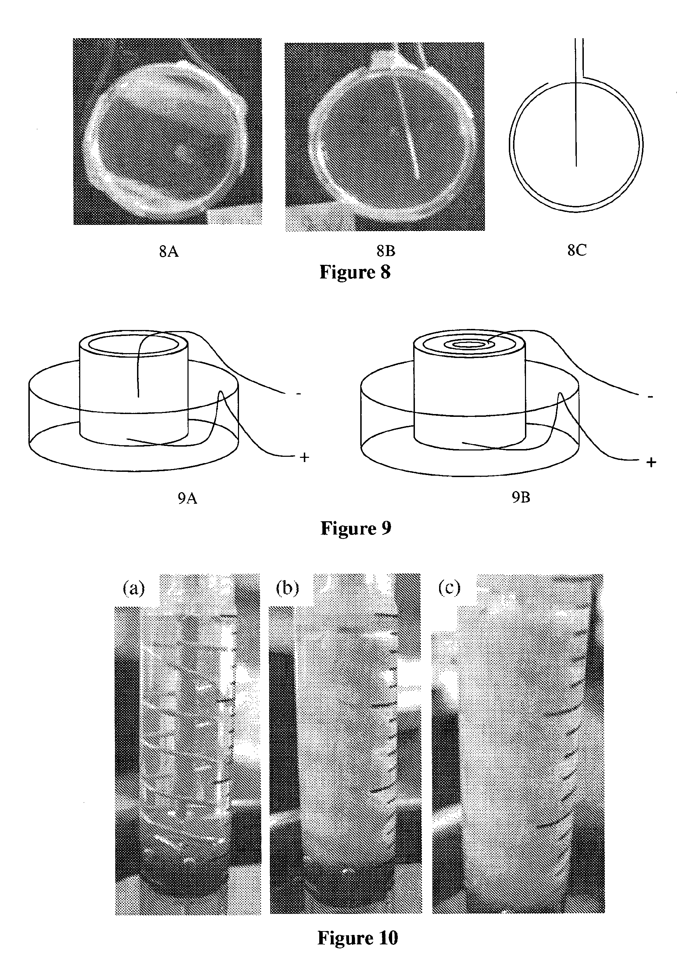

The methods in accordance with the present embodiments demonstrated that an applied electric field results in gelation of solubilized silk. The magnetic field induced by the applied electrical field is not a true cause of gelation, however. To demonstrate the magnetic field effect, a variation in the electrogelation setup was used. A positive ring-shaped electrode, best shown in Figure 8C, was formed by wrapping a bare wire around the outside of a culture dish, while a straight wire was used as a negative electrode. The negative electrode was submerged within a ∼9% silk solution, in the middle of the culture dish. To gauge the rate of gelation, a second experiment was conducted simultaneously using the parallel electrode setup used previously, shown in Figure 8A as a contol. Both experiments were conducted with a voltage potential of 24.8 VDC. As shown in Figure 8B, even after 30 min, no visible gelation occurred using the ring-shaped electrode setup (magnetic field). The significant gelation that occurred in the parallel-electrode setup confirmed that the lack of gelation in the ring-shaped electrode setup was not due to issues with the silk, electrode materials, or voltage applied.

### Example 5. Aluminum tube-based electrode

Successful electrogelation was achieved with a ring-shaped electrode submerged within a culture dish. This configuration provided evidence that increased electrode surface area could lead to greater electrogelation rates and total volume. Additionally, aluminum tubes can be used as electrodes. In one embodiment, an aluminum tube, acting as a positive electrode, was mounted vertically inside a culture dish using hot glue. An exposed wire, acting as a negative electrode, was positioned concentrically with the cylinder. A schematic illustration of this experimental setup is shown in Figure 9A. Observations from beneath the culture dish, comparing electrogelation times of 0 min, 7.5 min, and 15 min showed that electrogelation occurred quickly with this configuration, and resulted in conversion of at least 80% of the silk solution into gel.

To further understand the effect that electrode surface area has on electrogelation, another embodiment utilized two concentric aluminum tubes as electrodes. A schematic of this setup is shown in Figure 9B. Both tubes were hot glued to the bottom of a culture dish,
with ∼9% solubilized silk filling the annular volume. This experiment resulted in very fast gelation dynamics, producing rapid gelation and bubble formation. Nearly 100% of the annular volume was gelated. Both tube electrode embodiments clearly demonstrate that maximizing electrode surface area is one key to increasing electrogelation rate and final gelation volume.

### Example 6. Syringe-based metallized (foil) plunger electrode experiments

Given the success of the aluminum tube-based electrode embodiments, a separate embodiment utilized a syringe to both contain the silk solution and provide the necessary cylindrical electrode geometry to mimic the result of the aluminum tube-based electrode embodiment. In some syringe embodiments, the syringes were mounted vertically using standard lab stand hardware. In an initial design, aluminum foil was used to metallize the plunger of a 3 ml syringe. A syringe needle was inverted and positioned through the syringe nozzle, acting as the other electrode. Testing configurations were executed with the foil as the positive electrode or needle the negative electrode and vice versa. In either configuration, the electrogelation results were somewhat poor. This may be attribute to the limited foil surface area or poor electrical conductivity of the foil.

In an effort to improve electrogelation results with an aluminum foil-coated plunger, a 10 ml syringe plunger was modified. In this setup, the larger surface area of the plunger would be exploited to understand the effect of exposed area of the electrode. Very little electrogelation was witnessed, even after 30 min of voltage application.

### Example 7. Syringe-based metallized (plug) plunger electrode

Another syringe design substituted a 3/8" diameter aluminum plug for the aluminum foil. This plug was mounted into the plunger, with about 1/4" of the length extended into the silk solution volume. A syringe needle was passed into the solution from the nozzle end of the syringe. As in aluminum foil embodiments, this design was tested in two configurations: with the plug as the positive electrode and needle the negative and vice versa. With the needle as the positive electrode, more gelation was evidenced. The gelation process was slow for both configurations, however. The limited surface area of both plug and needle is likely the reason.

### Example 8. Syringe-based spring and needle electrode

A spring-shaped electrode geometry was constructed in another embodiment of the present application. A conducting wire was passed from the plunger side of the syringe through the plunger head. The wire, stripped of its insulation past the plunger head, was helically coiled to provide a significant number of turns within the syringe. The coil was designated the positive electrode. A needle was employed again as a negative electrode, with special attention paid to ensure no contact would be made between the positive and negative electrodes. Figures 10A-10C show the results of the electrogelation experiment after 0 min, 5 min, and 15 min, respectively. With the spring-shaped electrode acting as the positive electrode, very fast gelation was achieved, with almost 90% gel volume achieved after 15 min. This configuration is especially important due to the fact that the design does not hinder the extrusion of the gel from the syringe directly. When the syringe plunger is depressed, the spring-shaped electrode compresses, forcing the gel out of the syringe body. The gel was extruded from the syringe as a sticky, coherent stream, even though a syringe needle was not used. The use of a syringe needle can increase the gel stiffness further due to flow-induced shear.

### Example 9. Parallel electrode experiment comparing new (13 days) to older (65 days) solubilized silk

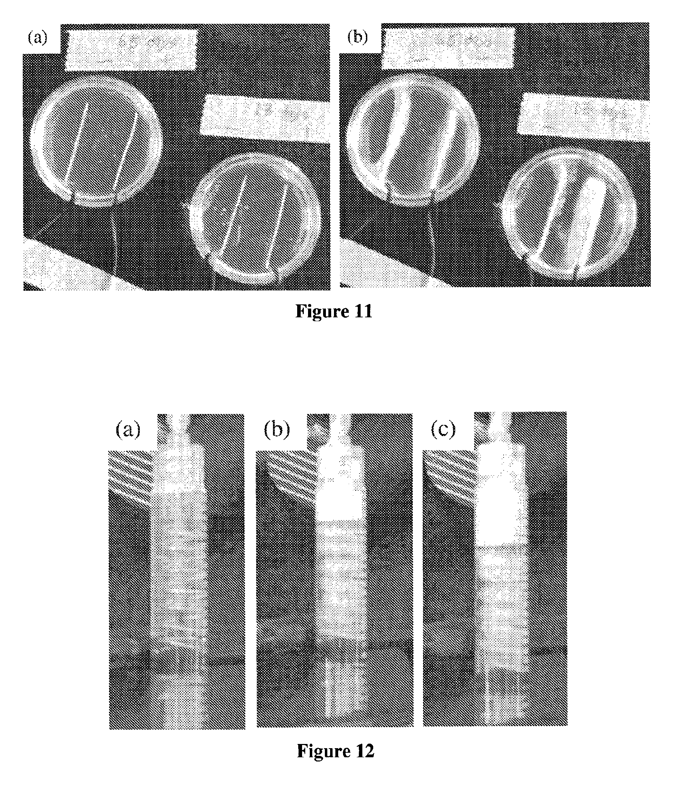

After silk is solubilized, it can eventually self-assemble over time. In self-assembly, conversion of the random coil nature of the silk solution is induced into conversion to β-sheet content, creating a stiff solid. Certain conditions, such as elevated pH and temperature, can accelerate the solidification. Using the parallel electrode configuration shown in Figure 4 with 24.8 VDC, a comparison was made between silk that had been prepared 13 days compared with silk that had been prepared 65 days prior to the test. Figure 11 shows the results after 0 min and 10 min of voltage application. While the older silk solution generated a larger volume of bubbles on the negative electrode, gelation appears to be faster in the newer silk.

### Example 10. Parallel electrode experiment comparing oxidized to non-oxidized electrode wire

To compare electrogelation rates as a function of oxidation, two simultaneous parallel electrode embodiments were constructed: one with electrodes that had been exposed to air for a prolonged period, the other with freshly exposed wire as electrodes. Only subtle differences were observed in this experiment. There was a brightness gradient in the gelated silk with the non-oxidized wire, while there was mostly bright white color throughout the gelated silk produced with the oxidized wire. It is possible that the brightness gradient occurs because the gelation is progressing faster using the non-oxidized wire. To confirm this observation, *in situ* electrical resistance and current measurements are made.

### Example 11. Three (3) ml Syringe-Based Spring and Needle

A practical gelation delivery tool was fashioned on the syringe-based spring and needle concept. A 3 ml syringe is a relatively small size that provides nearly complete electrogelation within a 10 min timeframe. Figure 12 shows that nearly 90% gelation was achieved over 10 min of voltage application. The resulting material could be readily ejected from the syringe in a coherent stream using the plunger. One interesting observation is that as bubbles formed at the top of the silk solution, enough pressure built up to push the syringe plunger downward.

### Example 12. Electrogelation using parallel copper coil electrode

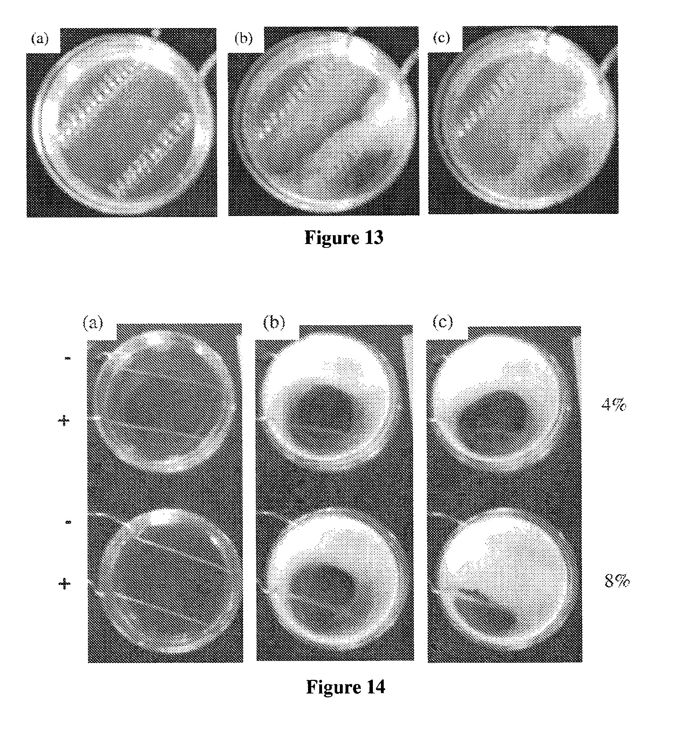

The electrogelation process involves an electrochemical reaction that is produced by an electrochemical cell including a DC voltage source, metallic positive and negative electrodes, and the solubilized silk solution. Gelation via parallel copper electrodes in solubilized silk confirmed the observations in other embodiments related to color change, bubble formation, and minor pitting of electrode metal; outcomes that can be associated with the proposed electrogelation mechanism. To provide enhanced gelation, both electrodes were formed into spring-like coils, increasing the surface area of both the positive and negative electrodes. The experimental results are shown in Figure 13. The electrogelation process was highly reactive, generating significant bubble formation at the negative electrode and overall gelation after 10 min of voltage application. A fairly distinct green-blue-indigo-violet color spectrum was generated, with green at the positive electrode and violet at the negative electrode.

### Example 13. Parallel electrode gelation of solubilized silk with salt and DMEM

For applications of electrogelation to tissue engineering, it is important to understand the ability to create viable constructs that can provide the proper structural integrity and cell environment. In one proposed scaffold geometry, solubilized silk is combined with salt and DMEM (Dulbecco's modified Eagle's medium) and solidified. After soaking in water, the dissolved salt leaves an ideal porous geometry. A series of tests were conducted to explore the possibility that electrogelation can be used in the solidification of such scaffold materials. As shown in Figure 14, parallel electrode experiments were conducted with salt- and DMEM-containing 4% and 8% solubilized silk. The DMEM gives the initial material a strong magenta color. During voltage application, an extreme amount of gaseous bubbles formed on the negative electrodes, with very little evidence of gelation on the positive. A strong green-blue coloration occurred, and after a short period of voltage application, the culture dish became too hot to touch. In addition, significant pitting of the positive electrode was apparent.

### Example 14. Syringe-based spring and needle electrode gelation of solubilized silk with salt and DMEM

The high temperatures generated during the parallel electrode experiments with the solubilized silk, salt and DMEM might reflect lower electrical resistance of the solution. The subsequent increase in current flow may make the process much more reactive. To investigate the ability to control the temperatures, a syringe-based version of the prior experiment has been conducted. The solubilized silk with the additives was placed in a 3 ml syringe, with a spring-shaped positive electrode and inverted needle used as the negative. The experiment was conducted in a 5°C refrigerator. The gelation process was very reactive, generating extreme amounts of gaseous bubbles within 90 sec, eventually almost completely evacuating the syringe of silk. No visible silk gelation occurred, although a color change to dark brown was witnessed.

### Example 15. Electrogelation experiments using graphite electrodes

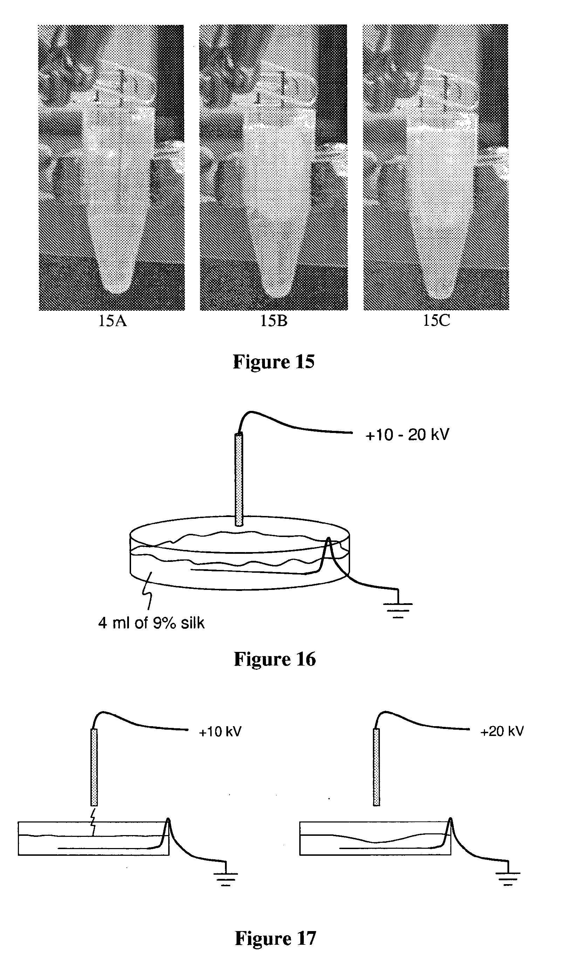

Electrogelation tests show that the metallic electrodes used play a key role in driving the reaction rate. Especially when using solutions with low electrical resistance, it can be important to select electrode materials that are more noble; that is, materials that have lower electrochemical potential. In this context, experiments were conducted using graphite electrodes made from mechanical pencil leads as both positive and negative electrodes. As shown in Figure 15, graphite makes very good electrodes, leading to rapid gelation, with a very well-defined three-dimensional gelation front. After just 10 min of voltage application, nearly the entire solubilized silk volume was gelated. Despite this fast and complete gelation, the gaseous bubble formation on the negative electrode was not excessive.

### Example 16. Low voltage (4 VDC) electrogelation experiments using graphite electrodes and solubilized silk with salt and DMEM

In the prior pencil lead experiment, the use of a more noble material appears to have stabilized the gelation process. Of course, graphite pencil leads are not made of pure carbon; nevertheless, there was a reduction in the gaseous bubble formation and the gelation front was well defined. Another experiment was conducted using this setup with solubilized silk containing salt and DMEM. To further keep the gelation process under control, a voltage of 4 VDC was applied. This approach still did not provide discernable gelation of the silk with additives, even after 8 min of voltage application. Fairly significant bubble formation occurred. In addition, a slight color change from the magenta to orange/brown was witnessed. The DMEM contains a marker that turns the color from magenta to yellow when the pH drops to about pH 6. This is likely the cause of the color change.

### Example 17. High voltage (25 VDC) electrogelation using pencil lead electrodes and solubilized silk with salt and DMEM

Prior experiments demonstrated that graphite electrodes maintained at low voltage can aid in controlling electrogelation processes. An experiment was conducted to understand the response of a pencil lead-based setup under high applied voltage. An applied voltage of 25 VDC caused a completely uncontrolled reaction in solubilized silk containing salt and DMEM. Most of the silk was displaced from the container by gaseous bubbles forming at the negative electrode and the rapid reaction created enough heat that the container was too hot to touch. No visible gelation was witnessed, although a hard, greenish residue was left behind in the sample container. This residue appeared to be precipitated solids in crystal form. Regardless of the electrode material, whether copper or carbon-based lead, it appears that high salt-content silk can be too conductive to allow a controlled gelation response in the silk without adjusting voltage and electrode efficiency.

### Example 18. Monitored electrogelation using graphite electrodes

Previous experiments demonstrated excellent electrogelation results when graphite electrodes were used on solubilized silk. To more carefully measure the response of the electrogelation, a controlled graphite electrode (mechanical pencil lead) experiment was conducted. Using 25 VDC and monitoring the current draw with an ammeter generated rapid gelation occurred over a span of 10 min, producing no color change, but a well-formed gelation front that resulted in almost complete gelation of the silk volume. The current data indicated that immediately when the voltage is applied, about 7 mA is drawn from the battery. This level drops nonlinearly until a steady-state current of 3 mA is drawn. This current draw trend likely parallels the growth trend of the silk gelation.

### Example 19. Electrogelation of silk-coated raw chicken

An important application for electrogelation is its application or administration to tissues. For example, electrogelation applied to skin might be employed to promote burn healing. An experiment was conducted as a proof-of-concept to see if raw chicken could be used to generate a silk-based gelation layer. A narrow strip of raw chicken was soaked in solubilized silk, acting as the positive electrode. A bare wire was used as the negative electrode. Upon applying a voltage, bubbles formed at the negative electrode, confirming that electrogelation was taking place. A lack of visual contrast between the semi-transparent silk gel and light-colored chicken tissue obscured visual results.

### Example 20. High voltage electrogelation of solubilized silk

To explore the effect of high voltage levels on electrogelation, a series of experiments were conducted using a high voltage power supply from Gamma High Voltage. An example embodiment is shown in Figure 16. A syringe needle was suspended 1 inch above the silk solution surface. The needle was set to either 10 kV or 20 kV positive potential. A bare wire electrode, submerged in the silk solution, was grounded. With the needle at +10 kV, significant sparks occurred, generated by the buildup of electrons at the needle tip and periodic discharge to the solution. No gelation was evident within the silk, although small bubbles were generated with each discharge. The cycling of the electron charging and discharging was controlled by the current-controlled power supply. With such a supply, the current draw is limited to prevent damage to the supply as the power level nears a peak allowable level. When the needle potential was increased to the +20 kV level, a significantly different response was witnessed. Instead of sparking and discharging of electrons, the silk solution directly under the needle pushed away from the needle position. The silk solution may have developed a positive charge on the top surface that was repelled by the needle. At this voltage level, the repulsion force caused the liquid volume to be manipulated. A follow-up experiment replicated this response. As the initial charge was applied, significant sparking occurred, with high current being drawn from the DC power supply. With adjustment of sample height and voltage, a stable trough in the silk solution was created. With decreased space between the solution and needle, the trough was driven deep enough that the bottom of the culture dish was nearly exposed. An additional observation with ambient lighting turned off, showed that a distinct blue halo was produced at the needle tip during the experiments. Upon further literature review, this appears to be a fairly well-known corona effect. The mechanism of creating the trough is likely attributable to ion wind - basically pumping ions at a high rate, causing the surface depression.

### Example 21. Device design - use of mixing to increase processing speed

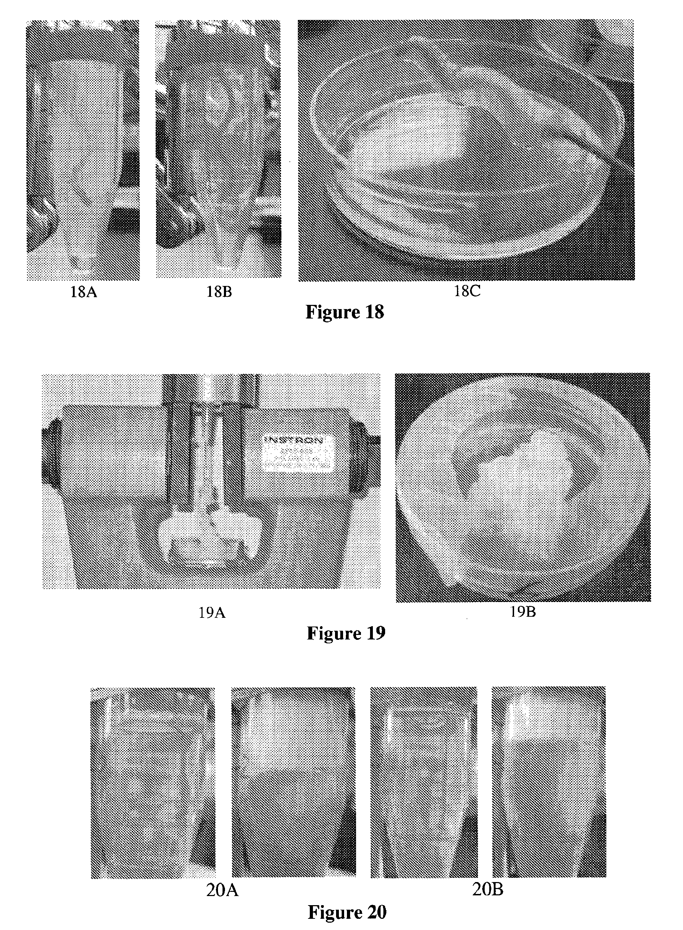

The original goal of these experiments was to focus a design effort on making an actual device that could produce electrogelated silk fast enough for real-time (continuous) usage. It is known that older silk can electrogelate faster than young (it is also known that after a certain age, the rate of electrogelation decreases - indicating that there is some limit to the positive effect of age on electrogelation rate). One key effect of time on silk is that self-assembly occurs, providing an increase in beta-sheet content. It was speculated that the shearing action of silk mixing could generate a similar effect; increasing beta-sheet content, like in older silk. An experiment was conducted with used 6 ml of 8.4% solubilized silk (32 days old) with 25 VDC and a conventional graphite electrode setup (0.5 mm type 2B pencil lead). The chamber was created by cutting a 10 ml Falcon tube in half (height-wise). A mixing auger was created by bending an insulated 22-gauge wire in one plane. The auger was mounted to a low RPM servo motor (10-15 RPM), controlled by a Parallax USB Servo board and a PC running Parallax Servo Controller Interface software version 0.9h Beta. As shown in Figure 18, the auger was positioned between the positive and negative electrodes, which are approximately 12 mm apart.

Over approximately the first 2 min of electrogelation, a solid would form on the positive pencil lead, as seen in prior experiments. As the solid grew in size, however, there was a transition during which the solid was pulled off of the positive electrode and became trapped on the auger as it was spinning. Once this transition occurred, the positive pencil lead would continue to generate silk gel, although it was continuously pulled onto the auger. After completion of the experiment, when the auger was removed from the Falcon tube, all of the silk gel remained on the auger (Figure 18C). Some distinct differences were observed with this silk gel: The color of the gel was strikingly clear, in contrast to the whitish color of prior gel. The gel was not as stiff as prior gels, but was very sticky, and a coherent solid, which could withstand some deformation. After depositing this gel on a surface at room temperature, it was observed to begin returning to a liquid form. Nearly complete reversal (80%-90%) to a liquid occurred after five days. After fifteen days, this percentage was observed to be about the same; no further transition back to a liquid appeared to have occurred. It was speculated that it is the presence of bubbles in the gel that are driving the reversal back to a liquid. It is important to note that as the auger was pulling the gel from the positive electrode, it also was pulling the bubbles from the negative electrode. The resulting rotating mass contained both gel and a lot of bubbles. The pH of the silk gel/bubble combination appeared nearly neutral: around pH 7.0 - pH 7.4. For applications in which quick gelation of a very sticky silk is desired and in which reversal back to liquid form is an advantage, this configuration is very promising. For example, this mechanism may be ideal for party string.

### Example 22. Use of mixing to increase processing speed (higher RPM)

Another mixing experiment was conducted using the same experimental parameters as Example 21 (8.4% silk, 33 days old, 0.5 mm 2B pencil lead, 25 VDC), except that only 4 ml of silk was used and a higher auger rotation rate (∼400 RPM) was used. After the test, it was observed that about 30% of the silk had been electrogelated and was adhering to the auger. The silk gel appeared to have increased stiffness than in the lower rotaton rate experiment.

### Example 23. Instron^{®} device-based extrusion testing of electrogelated silk

The stiffness of electrogelated silk was quantified by a series of mechanical tests. For most of the physical devices that would use electrogelated silk, it is likely that the silk would be extruded (pushed) out of an orifice. Therefore, the force required for extrusion and the mechanical properties of the extruded silk were studied. Applying shearing forces to solubilized silk causes beta-sheet formation, which increased the mechanical response of the silk. A very simple preliminary setup was used: the 8.4% concentration solubilized silk was electrogelated using a "standard" pencil lead embodiment. The electrogelated silk (about 2 ml) was removed from the experimental tube and inserted into a 5 ml syringe. The test was conducted on an Instron 3366 10-kN load frame with a 1 kN capacity load cell. A uniaxial compression method in BlueHill 2.0 was executed, which would push the silk out of the vertically-oriented syringe at a constant rate of 1 mm/second. As seen in Figure 19, the silk was extruded as a very stiff, coherent stream. The force response was recorded on separate graphs. The act of forcing the silk through the small needle provided a dramatic increase in the material stiffness. The resulting silk was also very sticky, as the exiting silk stream would tend to cluster into a ball and tend to stick to the needle.

This experiment was repeated after thirty days to quantify the effect of aging on the extrusion process. During the next extrusion test, the forces required to extrude the older silk out of the syringe needle were so great that the internal silk pressures resulting in syringe damage and not extrusion. Hence it appears that under some circumstances aging has an effect on the ability to generate a stiff silk using shearing (related to β-sheet content).

### Example 24. Voltage generation

Earlier experiments indicated that the silk solution acts like an electrochemical cell after electrogelation is begun. In other words, when the voltage was removed from the electrodes, a voltmeter would indicate a non-trivial level of DC voltage. To explore the ability of silk to act as an electrochemical cell, a series of experiments were conducted. The first baseline experiment involved submerging new mechanical pencil lead electrodes into a non-electrogelated batch of silk (4 ml of ∼8.4% silk, thirty-four days old) and recording any voltage present across them. Surprisingly, without ever having introduced an external voltage to this silk, a voltmeter indicated than 0.2 VDC was being generated. The anticipated current draw was likely in the micro-ampere range. It is speculated that the voltage generation could be due to the experiment acting as an electrochemical cell. Basically, the mechanical pencil lead is known to contain many materials beyond graphite, including metals. An electrochemical cell is typically set up by introducing electrodes of different material composition to an electrolyte. It is possible that the silk is acting as an electrolyte and the pencil lead has enough non-graphite metal, that it was acting like a battery. It is unknown at this time what determines which electrode is the positive or negative lead.

### Example 25. Electrode material influence on electrogelation

The use of graphite (mechanical pencil lead) as an electrode material was effective in electrogelation. The originally shiny surface of the electrode became rough after use in electrogelation. Knowing that the rate of electrogelation slows after initiation, it was speculated that the electrogelation could be largely driven by surface properties of the lead and not the bulk properties. To understand the effects of electrode degradation, a series of experiments were conducted. For each of these tests, new mechanical pencil lead electrodes were first used to electrogelate ∼8.4% silk (25 VDC for 10 min). After electrogelation, whatever solid had formed on the positive electrode was removed. As discussed above, the portion of each electrode that had been submerged in silk during electrogelation was left with a roughened surface. These used electrodes were then subsequently submerged back into the remaining solubilized silk, using the configurations shown below. Using a voltmeter attached to the electrodes, the voltage generated by the silk was measured. Whether the shiny half or roughened half was submerged during the voltage recording and which electrode was used as a positive/negative. One consistent result of the experiment is that when the positive electrodes taken from electrogelation was submerged for the voltage reading, a relatively large voltage was recorded (∼0.6 VDC). When the negative electrode was submerged, results were less consistent (possibly due to a change in ion kinetics). It is known that one influence on ion kinetics is solution pH, which may be dramatically affected by voltage applied in electrogelation.

Depending upon the electrogelation embodiment, while electrogelation is occurring the silk surrounding the positive electrode may have a lower pH than the virgin silk. After removal of the electrodes for use in the voltage measurement, the electrodes themselves will likely have pH values that reflect this behavior. When reintroduced into silk for voltage measurement, the silk likely continues to act as an electrochemical cell. The cell kinetics will be modified, depending upon whether the electrode pH enhances or suppresses the process. By submerging the positive electrode, which has a lower pH, the kinetics increase, producing a higher voltage reading. When the negative is submerged, which has an elevated pH, the kinetics are suppressed.

Possible physics behind electrogelation and implications for interpreting results warrant further discussion. When two different materials are either brought into direct contact or are brought into electrical contact through submersion in an electrolyte, a galvanic couple is created. Metals can have very different susceptibility to corrosion. Some metals, like zinc, can be very readily corroded, while others, most notably stainless steels and platinum, are corrosion-resistant. This property of metals is dictated by it's reaction in a galvanic couple. Less corrosive metals are more noble; that is, in a list of the galvanic series, these metals have a higher electrode potential. When the galvanic couple is created, the less noble metal will lose electrons which go into solution as metal cations. If a voltmeter were used to measure the potential between electrodes, the electrode made of the less noble metal could be considered the positive electrode and therefore the voltmeter would read a positive voltage potential. Electrons released will flow through the electrolyte to the more noble metal. On the surface of the more noble metal, dissolved oxygen will be reduced to hydroxide anions through the gaining of the electrons. If the less noble metal were not present, the other metal would still undergo reduction of oxygen to hydroxide, except the electrons would be furnished by this metal through oxidation.

Some of the electrode materials that have been used throughout the entire set of electrogelation experiments include platinum, copper, aluminum, and graphite. Platinum is very noble, while copper and aluminum are not (very low within the galvanic series). Although the main constituent in mechanical pencil lead is graphite, which is the most noble metal, there are other elements that are present. Unfortunately, it is therefore likely that the actual nobility of the graphite mechanical pencil lead is dependent not just on the graphite content, but the other elements as well. It is unknown what the actual composition of the graphite electrode used in these experiments is, or what is its corresponding electrochemical potential.

If electrodes of the same pure metal are used, a galvanic couple should not be created. From this point of view, unless another source of electrons is provided, no voltage potential should be measured across the electrodes. In the use of the aluminum, copper, and graphite electrodes in these electrogelation experiments, there are likely alloying elements and other material constituents that would cause a galvanic couple to be created. If one measures the voltage potential across the electrodes, the measurement would be nonzero and the voltage would be either positive or negative depending upon which electrode was contacted with the positive voltmeter lead. There appears to be an additional electrochemical response generated in the electrogelation process beyond the creation of a galvanic couple. Furthermore, silk is an unusual electrolyte. It is known that silkworm silk contains a variety of metal ions, which may remain in the solubilized silk solution after processing. It appears that these ions may influence the electrochemical process of electrogelation and are likely detected during voltmeter readings across electrodes.

### Example 26. Effects of platinum electrodes on electrogelation

In light of the discussion above, a series of experiments were undertaken to confirm that if a very noble, pure metal were used as electrodes, that the electrogelation would either not occur or would be much slower than if mechanical pencil leads were used. Using the frequently used experimental setup of Example 20, two electrodes were partially submerged in ∼8% solubilized silk fibroin (48-days-old). For these experiments, both mechanical pencil lead and 0.02" diameter, 99.95% pure platinum (Pt) electrodes were used. In addition, pH paper was used to record the pH of the silk solution before and after electrogelation. The 48-day-old silk solution was found to have a pH of pH 6.0 - pH 6.3. In the first experiment, a Pt electrode was used as a positive electrode, with a mechanical pencil lead negative electrode. With 25 VDC applied, electrogelation occurred at a rate that was significantly lower than if two pencil lead electrodes were used. Based on a visual observation from the power supply used, 2 mA of current was drawn during the experiment. After 10 min of electrogelation and the applied voltage was stopped, a voltage potential of 0.95 VDC was measured across the electrodes. This implies that the silk after electrogelation is acting like a battery; i.e., a DC voltage source. The resulting electrogelated silk was nearly transparent. Once the solid gel was removed from the non-electrogelated silk solution, the remaining solution was shown to have a pH 7.2 - pH 7.6. The pH of the solid gel surface (the pH paper could not be inserted into the interior of the solid) was found to be pH 4.9 - pH 5.1. The expected outcome of this experiment was that no electrogelation would occur, given the high nobility of Pt which was used as the positive electrode. While the gelation rate was slower, it clearly was occurring. As discussed herein, it is possible that the ions dispersed in the silk solution may have contributed to the electrochemical process that did occur (although one would expect the supply of electrons to be dissipated fairly quickly). Further, despite the fact that Pt is very corrosion-resistant, every metal does corrode at some level. Therefore, this very low corrosion rate could be contributing toward electrogelation.

Because both the positive and negative electrodes play a role in the galvanic corrosion explanation for electrogelation, a follow-up experiment was conducted to see which electrode plays the dominant role. The prior experiment was simply adjusted by using the mechanical pencil lead as the positive electrode and the Pt as the negative. All other experimental conditions remained the same. Electrogelation occurred at a rate that matched the rates observed when two mechanical pencil leads were used as electrodes. It appeared that the positive electrode plays the dominant role in dictating electrogelation rate. The resulting electrogelated silk was opaque and white (similar to the outcome from graphite electrodes). The remaining silk solution was found to have pH 9.4 - pH 9.7, while the electrogelated silk had pH 4.6 - pH 4.9. The measured voltage potential after gelation of 1.83 VDC is about double the voltage level observed when Pt was the positive electrode and graphite was the negative electrode. This voltage increase might be due to the increased number of electrons that would be ejected from the graphite electrode because it is less noble than the Pt and is much more susceptible to corrosion.

When two Pt electrodes were used for electrogelation, it was observed that electrogelation occurred, albeit at a slow rate. The rate was approximately the same as when the positive electrode was Pt and the negative was graphite. Interestingly, the resulting solid gel was observed to be an opaque white, not clear like the resulting gel in the experiment using a positive Pt electrode. The voltage potential across the Pt electrodes after electrogelation was measured to be 1.98 VDC. This is not consistent with the speculation that this "battery" potential is related to the amount of electrons pumped into the silk solution through a corrosion process. It is unknown what mechanism is causing the voltage increase and why transparency of the silk gel is influenced by the electrode combination. The remaining silk solution was found to have a pH of pH 8.0 - pH 8.5, while the electrogelated silk had a pH of pH 4.6 - pH 4.9.

### Example 27. Platinum electrodes with solubilized silk and DMEM

Prior experiments showed that the introduction of DMEM solution containing salt had a dramatic effect on electrogelation. Because the use of a Pt positive electrode still resulted in electrogelation while dramatically reducing the amount of corrosion that occurred, it was thought that the addition of DMEM would have a positive effect on the gelation rate, without contributing significantly to greater corrosion. Furthermore, the pH of the ungelated silk solution and the resulting gel is effected by electrogelation. It was also observed that the pH of DMEM was greater than the pH of solubilized silk. This elevated pH was speculated to be able to increase gelation rate when mixed with the solubilized silk before electrogelation. Experiments were conducted to observe the influence of DMEM on electrogelation rate when two Pt electrodes are used.

The DMEM and solubilized silk used in these experiments was observed to have pHs of pH 8.2 - pH 8.5 and pH 6.0 - pH 6.3, respectively. When mixed with a DMEM concentration of 40%, the resulting solution had a pH of pH 7.9 - pH 8.2. Using 25 VDC, the two Pt electrodes resulted in extremely fast gelation (∼1 min to achieve 90%-95% gel). Along with the fast forming of a solid on the positive electrode, very significant bubble formation occurred on the negative Pt electrode. The initially light purple coloration of the DMEM was changed to a light yellow where the silk electrogelated. The DMEM has a pH marker included that transitions to yellow when a pH of about pH 6 is reached. The non-electrogelated silk was observed have a pH of pH 8.8 - pH 9.7, while the gel was observed to have a pH of pH 3.1 - pH 3.4. The presence of the DMEM had a effect on electrogelation. In this case, however, the use of Pt electrodes resulting in controlled gelation rates. Changes in pH accompany the dramatic gelation rate changes; pH of the liquid increases and of the solid decreases.

In another experiment, the same double Pt electrode setup with a DMEM and silk mixture was used, although a lower voltage of 5 VDC was applied. This experiment resulted in an electrogelation rate that was about the same rate observed with graphite electrodes with an applied voltage of 25 VDC and solubilized silk only. After electrogelation of 10 min, about 90%-95% gelation was achieved. The resulting liquid silk and gel had pHs of pH 9.5 to pH 10.0 and pH 3.1 to pH 3.4, respectively.

### Example 28. Use of a dialysis cassette to isolate the electrodes and electrogelation products

One of the concerns of the electrogelation process is that the pH levels of the solution and gel can achieve extreme values (from pH 3 - pH 14). Another concern is that the process may involve electrolysis - the breaking down of the water content of the silk solution into hydrogen and oxygen gas, the hydrogen gas being problematic for cell survival. Therefore, an experiment was conducted using a dialysis cassette using a porous membrane to separate the products generated during electrogelation. A platinum electrode was inserted into the syringe port of a dialysis cassette filled with 8 wt% silk solution. This electrode was used initially as the positive electrode. A negative platinum electrode was immersed in de-ionized water containing common table salt. The application of 25 VDC to this electrogelation setup resulted in a good gelation response.

During this process, the hydrogen gas which typically forms on the negative electrode was kept outside of the dialysis cassette, away from the gel. After completion of the experiment, the pH of the un-gelated silk solution was shown to be between pH 7.9 and pH 8.2. This likely represents a slight pH rise over the pH of the virgin silk solution (for the 74-day-old silk solution used, the pH is likely about pH 6). The pH of the liquid on the surface of the gel was measured to be around pH 6 - pH 7.2. This experiment provides a method for keeping hydrogen gas segregated from the silk solution and gel by employing an appropriate membrane material. It is also clear that the pH range measured around the gel is much closer to neutral than in arrangements in which the positive and negative electrodes were submerged in the solubilized silk fibroin solution.

Electrogelation was also performed using a dialysis cassette setup, but with the negative electrode inside the cassette, positive outside, and using a 30 VDC supply voltage. Initially, the results were ambiguous: no visible gelation had occurred and the only visible response was the formation of bubbles on the electrodes. When the positive electrode was brought into contact with the dialysis cassette membrane, however, gel formed on the inside of the membrane along the area of contact of the electrode. Based on this result, the dialysis cassette was removed from the de-ionized water bath. Using the positive electrode like a writing instrument, it was possible to form shapes and letters on the inside surface of the membrane from silk gel. This result is intriguing because the cassette did not have to be in a bath and produced a controlled gelated geometry. This process might be exploited in the future to make a 3D, rapid prototyping tool using silk gel material. Application areas include the creation of customized silk-based tissue engineering scaffolds.

### Example 29. Reversed electrogelation - solidifying and re-liquifying silk gel

The silk gel microstructure generated in electrogelation may comprise primarily silk I structure (FTIR and CD measurements will confirm/refute this assertion). This meta-stable material phase is intriguing because it typically can be manipulated to go back to a random coil conformation (silk solution) or to form a β-sheet conformation (stiff gel and solid). Knowing that silk I can be re-liquified, an experiment was conducted to study this phenomenon as applied to electrogelation. Platinum electrodes were submerged in a 4 wt% silk solution. The electrodes were used to charge the solution with 25 VDC for 3 minutes. The outcome is shown in Figure 21B. The electrical connections were then reversed; what was a positive electrode was changed to be a negative electrode and the former negative electrode was made positive. The experimental outcome is shown in Figure 21C. The silk gel that had formed originally on the positive electrode became semi-transparent before entirely disappearing. A new gel formed on the electrode that was formerly the negative electrode, as shown. This experiment showed that the gelation process can be fully reversed. Electrogelation, therefore, shows promise as a process for applications in which a transition from liquid to solid is important. For example, it can be envisioned that electrogelation could be used to actuate a soft "leg" in a soft-bodied robot; cyclic solidification and re-liquification would cause locomotion.

The invention provides a method of converting a solubilized silk fibroin solution into a silk fibroin gel, the method comprising:
applying an electric field to said solubilized silk fibroin solution to create the silk fibroin gel.

Suitably, said electric field is applied using a DC voltage of about 20 VDC to about 75 VDC.

Typically, said electric field is applied using a high-current lithium polymer (Li-Pol) battery.

Preferably, the method further comprises:
converting the silk fibroin gel from silk I conformation to β-sheet structure,
wherein the converting includes at least one of dehydrating the silk fibroin gel, applying mechanical force to the silk fibroin gel, elevating PH of the silk fibroin gel, elevating temperature of the silk fibroin gel, and introducing salt to the silk fibroin gel.

Generally, the mechanical force is a flow-induced shear force created by extruding the silk fibroin gel through an orifice.

The invention also provides an electrogelated silk composition prepared according to the method of the inevention.

Suitably, the electrogelated silk is edible and further comprises a flavoring agent.

Typically, the electrogelated silk is delivered to a subject or location in the form of a spray, stream, slow extrusion, or bulk extraction.

Generally, the electrogelated silk is produced as a toy or a party decoration.

Typically, the electrogelated silk is produced as a flame retardant.

Suitably, the electrogelated silk is formulated as a burn or wound treatment.

Optionally, the electrogelated silk is formulated as a tissue regeneration composition.

Typically, the electrogelated silk is formulated as a medical filling material.

Generally, the electrogelated silk is produced as a machine component.

The invention also provides a piezoelectric silk material comprising:
a mixture of silk fibroin proteins having silk I conformation and silk β-sheet structure;
wherein the piezoelectric property of the silk material is characterized by the ratio of silk I conformation and silk β-sheet structure,
wherein the content of silk I conformation is adjusted by DC voltage and the content of silk β-sheet structure is adjusted by applying at least once of dehydration, mechanical force or thermodynamic treatment to the silk material.

The invention also provides a regenerated tissue material comprising the piezoelectric silk material of the invention.

Typically, the regenerated tissue material is at least one of a regenerated bone, tendon, dentin, aorta, trachea, or intestine material.

The invention also provides a mechanical device or component comprising the piezoelectric Silk material of the invention.

Typically, the mechanical device is at least one of an actuator, braking fluid, a valve component, or shock absorber material.

The invention also provides a device for processing and delivering electrogelated silk comprising:
a housing;
a power source removably disposed within said housing;
a pump in communication with said power source;
a container holding a solubilized silk fibroin solution, said container operably connected to said pump;
a positive electrode and negative electrode, each in communication with said power source and disposed to make contact with said container;
a controller configured to apply voltage to said electrodes from said power source to effect electrogelation of a silk fibroin solution to create a silk gel, wherein said controller also actuates said pump;
an activator for actuating said pump to eject silk gel from said device; and
a nozzle removably disposed in said housing through which the silk gel is ejected from said container.

Suitably, stiffness of the ejected silk gel is adjusted by the size of the aperture of said nozzle.

The device suitably further comprises a timing board or a signal display.

The invention also provides a method of encapsulating at least one agent in a silk fibroin gel, the method comprising:
introducing the at least one agent to a solubilized silk fibroin solution;
applying an electric field to the silk fibroin solution to induce electrogelation of the silk fibroin solution to form a silk fibroin gel-encapsulated active agent.

Typically, the at least one agent is a bioactive agent selected from the group consisting of cells, proteins, peptides, nucleic acid analogues, nucleotides or oligonucleotides, peptide nucleic acids, aptamers, antibodies or portions or fragments thereof, hormones, hormone antagonists, growth factors or recombinant growth factors and portions, fragments or variants thereof, cytokines, enzymes, antibiotics or antimicrobial compounds, viruses, antivirals, antifungals, toxins, prodrugs, chemotherapeutic agents, small molecules, drugs, and combinations thereof.

The invention also provides a bioactive agent-encapsulated silk fibroin gel, prepared according to the method of the invention.

The invention also provides a biodelivery device comprising the bioactive agent-encapsulated silk fibroin gel of the invention.

The invention also provides a pharmaceutical formulation comprising the bioactive agent-encapsulated silk fibroin gel of the invention and a pharmaceutically acceptable excipient. The invention also provides a method of coating at least part of a substrate with a silk fibroin gel comprising:
exposing said at least part of the substrate to a solubilized silk fibroin solution;
applying an electric field to said substrate, the substrate operably connected to a positive electrode to induce electrogelation of the silk fibroin solution;
accumulating a silk fibroin gel coating on the surface of the substrate.

Typically, the silk fibroin gel coating is further dehydrated to convert to a silk fibroin film,

Suitably, the substrate to be coated with the silk gel is a tissue, regenerated tissue, medical device, medical implant, veterinary device, or veterinary implant.

Generally, the method is used for in vivo tissue regeneration or tissue defect repair.

Typically, the tissue is a skin tissue.

Preferably, the regenerated tissue, medical device or implant is produced for use in a field selected from the group consisting of dentistry, orthopedics, traumatology, cardiovascular application, coagulation devices, implantable artificial organs, small implantation materials of medical instruments.

Ideally, the regenerated tissue, medical device or implant is selected from the group consisting of dental filling material dental prosthetic devices comprising crowns, bridges, removable prostheses; dental implants comprising osseointegrated implant; orthodontic appliances comprising dental braces and retainer; orthopaedic bone filling materials; arthroplasty devices or implants comprising hip and knee shoulder and elbow prostheses, bone cements, prosthetic joint components; maxillofacial implants and devices comprising skulk face, and jaw prostheses; spinal devices or implants comprising spinal cord, spinal column, spine cage; osteosynthesis devices and instrumentation for fixation of bones and/or joint components comprising nails, wires, pin, plates, rods, wires, screws or other fixator; wound healing devices of implants comprising skin, bone, and vascular wound grafts and patches, sutures, vascular wound repair devices, and hemostatic dressings, cardiovascular devices and implants comprising blood vessel prosthesis, stents, catheters, vascular grafts, heart valves and pacemakers, coagulation devices comprising argon enhanced coagulation devices with coated probes; implantable artificial organs comprising artificial heart, liver, kidney, lungs, brain pacemaker and the like; small implantation materials comprising occluders, filters, screws, bolts, nuts, pins, plates, gears, sleeve bearings, washers, anchors, plugs, nails and the like; and medical instruments comprising scalpels, forceps, scoops, currettes, steotomes, chisels, gouges, strippers and the like.

The method of the invention typically includes a step of embedding at least one active agent in the silk gel coating.

Suitably, the at least one agent is a bioactive agent selected from the group consisting of cells, proteins, peptides, nucleic acid analogues, nucleotides or oligonucleotides, peptide nucleic acids, aptamers, antibodies or fragments or portions thereof, hormones, hormone antagonists, growth factors or recombinant growth factors and fragments and variants thereof, cytokines, enzymes, antibiotics or antimicrobial compounds, viruses, antivirals, toxins, prodrugs, chemotherapeutic agents, small molecules, drugs, and combinations thereof.

Preferably, the method includes the step of embedding a cell growth media in the silk gel coating.

Typically, the regenerated tissue, medical device or implant is fabricated from a material selected from the group of metals, alloys, electrical conductive polymers or ceramics, or combination thereof.

The invention also provides a method of reversibly converting a solubilized silk fibroin solution into a silk fibroin gel comprising:
applying DC voltage to said silk fibroin solution to induce electrogelation; and
reversing the electrode polarity, whereupon the electrogelated silk fibroin gel is converted to silk fibroin solution.

The invention also provides a method of alternating of fluid viscosity, tackiness, and stiffness of the silk gel comprising applying and reversing the electrogelation method of the invention.

The invention provides a soft robotic device capable of motion along a medium, comprising a silk muco-adhesive, wherein said motion of the soft robotic device is regulated by controlling the tackiness of the silk muco-adhesive on the medium through applying and reversing the electrogelation method of the invention,

Suitably, the device is capable of adhering to the medium when the device is not in motion.

Typically, the device is capable of adhering to the medium when the medium is wet or in an aqueous environment,

Generally, the soft robotic device is used in combination with monitoring, delivery, diagnostic, or treating devices.

## Claims

1. A method of converting a solubilized silk fibroin solution into a silk fibroin gel, the method comprising:
applying an electric field to said solubilized silk fibroin solution to create the
silk fibroin gel,

2. An electrogelated silk composition prepared according to the method of claim 1.

3. A piezoelectric silk material comprising:
a mixture of silk fibroin proteins having silk I conformation and silk β-sheet structure;
wherein the piezoelectric property of the silk material is **characterized by** the ratio of silk I conformation and silk β-sheet structure;
wherein the content of silk 1 conformation is adjusted by DC voltage and the content of silk β-sheet structure is adjusted by applying at least one of dehydration, mechanical force or thermodynamic treatment to the silk material.

4. A regenerated tissue material comprising the piezoelectric silk material or claim 3.

5. A mechanical device or component comprising the piezoelectric silk material of claim 3.

6. A device for processing and delivering electrogelated silk comprising:
a housing;
a power source removably disposed within said housing;
a pump in communication with said power source;
a coutainer holding a solubilized silk fibroin solution, said container operably connected to said pump;
a positive electrode and negative electrode, each in communication with said power source and disposed to make contact with said container;
a controller configured to apply voltage to said electrodes from said power source to effect electrogelation of a silk fibroin solution to create a silk gel, wherein said controller also actuates said pump;
an activator for actuating said pump to eject silk gel from said device; and
a nozzle removably disposed in said housing through which the silk gel is ejected from said container.

7. A method of encapsulating at least one agent in a silk fibroin gel, the method comprising:
introducing the at least one agent to a solubilized silk fibroin solution;
applying an electric field to the silk fibroin solution to induce electrogelation of the silk fibroin solution to form a silk fibroin gel-encapsulated active agent.

8. An agent-encapsulated silk fibroin gel, prepared according to the method of claim 7, in which the agent is a bioactive agent selected from the group consisting of cells, proteins, peptides, nucleic acid analogues, nucleotides or oligonucleotides, peptide nucleic acids, aptamers, antibodies or portions or fragments thereof, hromones, hormone antagonists, growth factors or recombinant growth factors and portions, fragments or variants thereof, cytokines, enzymes, antibiotics or antimicrobial compounds, viruses, antivirals, antifungals, toxins, prodrugs, chemotherapeutic agents, small molecules, drugs and combinations thereof.

9. A biodelivery device comprising the bioactive agent-encapsulated silk fibroin gel of claim 8.

10. A pharmaceutical formulation comprising the bioactive agent-encapsulated silk fibroin gel of claim 8 and a pharmaceutically acceplable excipient.

11. A method of coating at least part of a substrate with a silk fibroin gel comprising:
exposing said at least part of the substrate to a solubilized silk fibroin solution;
applying an electric field to said substrate, the substrate operably connected to a positive electrode to induce electrogelation of the silk fibroin solution;
accumulating a silk fibroin gel coating on the surface of the substrate.

12. A method of reversibly converting a solubilized silk fibroin solution into a silk fibroin gel comprising:
applying DC voltage to said silk fibroin solution to induce electrogelation; and
reversing the electrode polarity, whereupon the electrogelated silk fibroin gei is converted to silk fibroin solution.

13. A method of alternating of fluid viscosity, tackiness, and stiffness of the silk gel comprising applying and reversing the electrogelation method as in claim 12.

14. A soft robotic device capable of motion along a medium, comprising a silk muco-adhesive, wherein said motion of the soft robotic device is regulated by controlling the tackiness of the silk muco-adhesive on the medium through applying and reversing the electrogelation method as in claim 12.

## Patentansprüche

1. Verfahren zum Umwandeln einer Lösung aus gelöstem Seidenfibroin in ein Seidenfibroingel, wobei das Verfahren Folgendes umfasst:
Anlegen eines elektrischen Felds an die Lösung aus gelöstem Seidenfibroin, um das Seidenfibroingel zu erzeugen.

2. Elektrogelierte Seidenzusammensetzung, die gemäß dem Verfahren von Anspruch 1 zubereitet wird.

3. Piezoelektrisches Seidenmaterial, das Folgendes umfasst:
ein Gemisch aus Seidenfibroinproteinen mit Seide I-Konformation und Seiden-β-Faltblattstruktur;
wobei die piezoelektrische Eigenschaft des Seidenmaterials **gekennzeichnet ist durch** das Verhältnis von Seide I-Konformation und Seiden-β-Faltblattstruktur;
wobei der Gehalt an Seide I-Konformation **durch** Gleichspannung angepasst wird und der Gehalt an Seiden-β-Faltblattstruktur **durch** Anwenden von mindestens einem von Dehydrierung, mechanischer Kraft oder
thermodynamischer Behandlung auf das Seidenmaterial angepasst wird.

4. Regeneriertes Gewebematerial, das das piezoelektrische Seidenmaterial nach Anspruch 3 umfasst.

5. Mechanische Vorrichtung oder Komponente, die das piezoelektrische Seidenmaterial nach Anspruch 3 umfasst.

6. Vorrichtung zum Verarbeiten und Verabreichen von elektrogelierter Seide, die Folgendes umfasst:
ein Gehäuse;
eine Leistungsquelle, die entfernbar in dem Gehäuse angeordnet ist;
eine mit der Leistungsquelle in Verbindung stehende Pumpe;
einen Behälter, der eine Lösung aus gelöstem Seidenfibroin enthält, wobei der Behälter wirksam mit der Pumpe verbunden ist;
eine positive Elektrode und eine negative Elektrode, die jeweils mit der Leistungsquelle in Verbindung stehen und dazu angeordnet sind, mit dem Behälter in Kontakt zu treten;
ein Steuergerät, das dazu ausgebildet ist, Spannung von der Leistungsquelle an die Elektroden anzulegen, um die Elektrogelierung einer Seidenfibroinlösung zu bewirken, um ein Seidengel zu erzeugen, wobei das Steuergerät außerdem die Pumpe betätigt;
einen Auslöser zum Betätigen der Pumpe, um Seidengel aus der Vorrichtung auszustoßen; und
eine entfernbar in dem Gehäuse angeordnete Düse, durch die das Seidengel aus dem Behälter ausgestoßen wird.

7. Verfahren zum Kapseln von mindestens einem Wirkstoff in einem Seidenfibroingel, wobei das Verfahren Folgendes umfasst:
Einbringen des mindestens einen Wirkstoffs in eine Lösung aus gelöstem Seidenfibroin;
Anlegen eines elektrischen Felds an die Seidenfibroinlösung, um die Elektrogelierung der Seidenfibroinlösung herbeizuführen, um einen in Seidenfibroingel gekapselten aktiven Wirkstoff zu bilden.

8. Wirkstoffgekapseltes Seidenfibroingel, das gemäß dem Verfahren von Anspruch 7 zubereitet wird, wobei es sich bei dem Wirkstoff um einen bioaktiven Wirkstoff handelt, der aus der Gruppe ausgewählt ist, die besteht aus: Zellen, Proteinen, Peptiden, Nukleinsäureanalogen, Nukleotiden oder Oligonukleotiden, Peptidnukleinsäuren, Aptameren, Antikörpern oder Anteilen oder Fragmenten derselben, Hormonen, Hormonantagonisten, Wachstumsfaktoren oder rekombinanten Wachstumsfaktoren und Anteilen, Fragmenten oder Varianten derselben, Cytokinen, Enzymen, Antibiotika oder antimikrobiellen Verbindungen, Viren, Viruziden, Fungiziden, Toxinen, Pro-Pharmaka, chemotherapeutischen Wirkstoffen, kleinen Molekülen, Arzneimitteln und Kombinationen derselben.

9. Bioverabreichungsvorrichtung, die das in bioaktivem Wirkstoff gekapselte Seidenfibroingel nach Anspruch 8 umfasst.

10. Pharmazeutische Formulierung, umfassend das in bioaktivem Wirkstoff gekapselte Seidenfibroingel nach Anspruch 8 und einen pharmazeutisch zulässigen Hilfsstoff.

11. Verfahren zum Beschichten von mindestens einem Teil eines Substrats mit einem Seidenfibroingel, das Folgendes umfasst:
Aussetzen von mindestens einem Teil des Substrats an eine Lösung aus gelöstem Seidenfibroin;
Anlegen eines elektrischen Felds an das Substrat, wobei das Substrat wirksam mit einer positiven Elektrode verbunden ist, um die Elektrogelierung der Seidenfibroinlösung herbeizuführen;
Akkumulieren einer Seidenfibroingelbeschichtung auf der Oberfläche des Substrats.

12. Verfahren zum umkehrbaren Umwandeln einer Lösung aus gelöstem Seidenfibroin in ein Seidenfibroingel, das Folgendes umfasst:
Anlegen von Gleichspannung an die Seidenfibroinlösung, um die Elektrogelierung herbeizuführen, und
Umkehren der Elektrodenpolarität, woraufhin das elektrogelierte Seidenfibroingel in eine Seidenfibroinlösung umgewandelt wird.

13. Verfahren zum Alternieren von Fluidviskosität, Klebrigkeit und Steifigkeit des Seidengels, umfassend das Anwenden und Umkehren des Elektrogelierungsverfahrens nach Anspruch 12.

14. Weichrobotische Vorrichtung, die zu Bewegung entlang einem Medium fähig ist, umfassend einen Seidenschleimkleber, wobei die Bewegung der weichrobotischen Vorrichtung durch Steuern der Klebrigkeit des Seidenschleimklebers auf dem Medium durch Anwenden und Umkehren des Elektrogelierungsverfahrens nach Anspruch 12 reguliert wird.

## Revendications

1. Un procédé de conversion d'une solution de fibroïne de soie solubilisée en un gel de fibroïne de soie, le procédé comprenant :
l'application d'un champ électrique à ladite solution de fibroïne de soie solubilisée de façon à créer le gel de fibroïne de soie.

2. Une composition de soie électrogélifiée préparée selon le procédé selon la Revendication 1.

3. Un matériau de soie piézoélectrique comprenant :
un mélange de protéines de fibroïne de soie possédant une conformation I de la soie et une structure de feuille β de la soie,
où la propriété piézoélectrique du matériau de soie est **caractérisée par** le rapport de la conformation I de la soie sur la structure de feuille β de la soie,
où la teneur de conformation I de la soie est ajustée par une tension de c.c. et la teneur de structure de feuille β de la soie est ajustée par l'application d'au moins un élément parmi déshydratation, force mécanique ou traitement thermodynamique au matériau de soie.

4. Un matériau tissulaire régénéré contenant le matériau de soie piézoélectrique selon la Revendication 3.

5. Un dispositif ou composant mécanique comprenant le matériau de soie piézoélectrique selon la Revendication 3.

6. Un dispositif de traitement et de fourniture de soie électrogélifiée comprenant :
un boîtier,
une source d'alimentation électrique disposée de manière amovible à l'intérieur dudit boîtier,
une pompe en communication avec ladite source d'alimentation électrique,
un récipient contenant une solution de fibroïne de soie solubilisée, ledit récipient étant raccordé de manière opérationnelle à ladite pompe,
une électrode positive et une électrode négative, chacune étant en communication avec ladite source d'alimentation électrique et étant disposée de façon à établir un contact avec ledit récipient,
un système de commande configuré de façon à appliquer une tension auxdites électrodes à partir de ladite source d'alimentation électrique de façon à réaliser une électrogélation d'une solution de fibroïne de soie de façon à créer un gel de soie, où ledit système de commande actionne également ladite pompe,
un activateur destiné à actionner ladite pompe de façon à éjecter du gel de soie à partir dudit dispositif, et
une buse disposée de manière amovible dans ledit boîtier au travers de laquelle le gel de soie est éjecté dudit récipient.

7. Un procédé d'encapsulation d'au moins un agent dans un gel de fibroïne de soie, le procédé comprenant :
l'introduction du au moins un agent dans une solution de fibroïne de soie solubilisée,
l'application d'un champ électrique à la solution de fibroïne de soie de façon à induire une électrogélation de la solution de fibroïne de soie de façon à former un agent actif encapsulé dans un gel de fibroïne de soie.

8. Un gel de fibroïne de soie encapsulé dans un agent préparé selon le procédé de la Revendication 7 dans lequel l'agent est un agent bioactif sélectionné dans le groupe se composant de cellules, protéines, peptides, analogues d'acides nucléiques, nucléotides ou oligonucléotides, acides nucléiques de peptide, aptamères, anticorps ou parties ou fragments de ceux-ci, hormones, antagonistes d'hormones, facteurs de croissance ou facteurs de croissance recombinants et parties, fragments ou variantes de ceux-ci, cytokines, enzymes, antibiotiques ou composés antimicrobiens, virus, antiviraux, antifongiques, toxines, promédicaments, agents chimiothérapeutiques, petites molécules, médicaments, et des combinaisons de ceux-ci.

9. Un dispositif de bioadministration comprenant le gel de fibroïne de soie encapsulé dans un agent bioactif selon la Revendication 8.

10. Une formulation pharmaceutique comprenant le gel de fibroïne de soie encapsulé dans un agent bioactif selon la Revendication 8 et un excipient pharmaceutiquement acceptable.

11. Un procédé de revêtement d'au moins partie d'un substrat avec un gel de fibroïne de soie comprenant :
l'exposition de ladite au moins partie du substrat à une solution de fibroïne de soie solubilisée,
l'application d'un champ électrique audit substrat, le substrat étant raccordé de manière opérationnelle à une électrode positive de façon à induire une électrogélation de la solution de fibroïne de soie,
le dépôt d'un revêtement de gel de fibroïne de soie sur la surface du substrat.

12. Un procédé de conversion réversible d'une solution de fibroïne de soie solubilisée en un gel de fibroïne de soie comprenant :
l'application d'une tension de c.c. à ladite solution de fibroïne de soie de façon à induire une électrogélation, et
l'inversion de la polarité de l'électrode, après quoi le gel de fibroïne de soie électrogélifié est converti en une solution de fibroïne de soie.

13. Un procédé visant à alterner viscosité de fluide, adhésivité et rigidité du gel de soie comprenant l'application et l'inversion du procédé d'électrogélation selon la Revendication 12.

14. Un dispositif robotique mou capable de se déplacer le long d'un milieu, contenant un muco-adhésif de soie, où ledit déplacement du dispositif robotique mou est régulé par la commande de l'adhésivité du muco-adhésif de soie sur le milieu par l'application et l'inversion du procédé d'électrogélation selon la Revendication 12.
